(19)

**Europäisches Patentamt**
**European Patent Office**
**Office européen des brevets**

(11) **EP 1 276 770 B1**

(12) **EUROPÄISCHE PATENTSCHRIFT**

(45) Veröffentlichungstag und Bekanntmachung des Hinweises auf die Patenterteilung:
**17.10.2007   Patentblatt 2007/42**

(21) Anmeldenummer: **01931566.2**

(22) Anmeldetag: **04.04.2001**

(51) Int Cl.:
*C07K 16/00* (2006.01)          *A61K 39/395* (2006.01)
*A61K 49/00* (2006.01)          *C12P 21/08* (2006.01)

(86) Internationale Anmeldenummer:
**PCT/EP2001/003867**

(87) Internationale Veröffentlichungsnummer:
**WO 2001/077179 (18.10.2001 Gazette 2001/42)**

(54) **PROTEINE MIT HOHER IMMUNREAKTIVITÄT SOWIE EIN VERFAHREN ZU IHRER HERSTELLUNG**

PROTEINS WITH A HIGH IMMUNOREACTIVITY AND A METHOD FOR THE PRODUCTION THEREOF

PROTEINES PRESENTANT UNE FORTE IMMUNOREACTIVITE ET LEUR PROCEDE DE PRODUCTION

(84) Benannte Vertragsstaaten:
**AT BE CH CY DE DK ES FI FR GB GR IE IT LI LU MC NL PT SE TR**

(30) Priorität: **05.04.2000   DE 10016877**

(43) Veröffentlichungstag der Anmeldung:
**22.01.2003   Patentblatt 2003/04**

(73) Patentinhaber: **Scintec Diagnostics Gmbh**
**6301 Zug (CH)**

(72) Erfinder:
 • **BENES, Ivan, F.**
 **CH-8127 Forch (CH)**
 • **THOMSEN-BOSSLET, Silke**
 **13465 Berlin (DE)**

(74) Vertreter: **Weiss, Wolfgang et al**
**Weickmann & Weickmann**
**Patentanwälte**
**Postfach 86 08 20**
**81635 München (DE)**

(56) Entgegenhaltungen:
**EP-A- 0 727 480          EP-B1- 0 585 570**
**WO-A-86/05202          DE-A- 19 521 388**

 • **BRUYNCK A ET AL: "CHARACTERISATION OF A HUMANISED BISPECIFIC MONOCLONAL ANTIBODY FOR CANCER THERAPY" BRITISH JOURNAL OF CANCER, LONDON, GB, Bd. 67, Nr. 3, 1993, Seiten 436-440, XP002901232 ISSN: 0007-0920**
 • **DUX R ET AL: "Determination of immunoreactive fraction and kinetic parameters of a radiolabeled monoclonal antibody in the absence of antigen excess." JOURNAL OF IMMUNOLOGICAL METHODS, (1991 NOV 22) 144 (2) 175-83. , XP002174108**
 • **SECCAMANI, E. ET AL: "Standardization of an immunoreactivity test for radiolabelled monoclonal antibodies" NUKLEARMEDIZIN, SUPPL. (STUTTGART) (1989), 25(TRENDS POSSIBILITIES NUCL. MED.), 709-11 , XP001015440**
 • **DATABASE CHEMABS [Online] CHEMICAL ABSTRACTS SERVICE, COLUMBUS, OHIO, US; GUO XUEGANG ET AL: "A method to determine the affinity constant of an antibody and antigen density on cell surfaces" retrieved from STN Database accession no. 121:202700 HCA XP002174109 & RECENT ADV. CHEM. MOL. BIOL. CANCER RES., INT. SYMP. (1993), MEETING DATE 1991, 161-6. EDITOR(S): DAI, QIANHUAN; ARMOUR, MARGARET-ANN; ZHENG, QINGYING. PUBLISHER: SCIENCE PRESS, BEIJING, PEOP. REP. CHINA. ,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft Präparationen von immunreaktiven Proteinen, vorzugsweise in aufgereinigter Form, die einen hohen Anteil an immunreaktiven Molekülen bezüglich der Gesamtzahl von Molekülen aufweisen. Diese Proteine sind durch Fermentation einer das immunreaktive Protein exprimierenden Wirtszelle in einem Wirbelschichtreaktor und Gewinnung des Proteins aus der Wirtszelle bzw. des zur Kultivierung verwendeten Mediums erhältlich. Die erfindungsgemäßen Proteinpräparationen sind hervorragend zur Herstellung diagnostischer und therapeutischer Zusammensetzungen geeignet.

Diagnostische und therapeutische Proteine können sowohl in prokaryontischen (z.B. E. coli (Houston et al., US-Patent 5,132,405)) als auch in eukaryontischen Zellsystemen (z.B. Pichia pastoris, Baby Hamster Kidney (BHK-) Zellen, Chinese Hamster Ovary (CHO-)Zellen, Hybridomen, transgenen Tieren und Pflanzen (Meade et al., US-Patent 4,873,316)) exprimiert und mittels proteinchemischer Verfahren gereinigt werden.

Prokaryontische Zellsysteme erlauben aufgrund der anspruchslosen Nährmedien und des schnellen Wachstums der Mikroorganismen in wenig anspruchsvollen Fermentationssystemen eine kostengünstige Herstellung von kleineren, Kohlenhydrat-freien Proteinen.

Für die Herstellung komplexer, Kohlenhydrat-tragender (Glyko-) Proteine müssen allerdings die oben erwähnten eukaryontischen Zellsysteme verwendet werden, die aufwendige Fermentationssysteme und die Verwendung von teuren Zellkulturmedien bedingen. Die Beschaffenheit der zu reinigenden (Glyko-) Protein-Präparationen wird dabei ganz entscheidend von dem eingesetzten Zellkulturmedium und dem verwendeten Fermentationssystem beeinflusst. Vor allem Unterschiede in der Glykosilierung des Proteins, die einen Einfluss auf die Halbwertzeit im Plasma sowie auf bestimmte biologische Effektorfunktionen der gereinigten (Glyko-) Protein-Präparation haben, sind in der Fachliteratur beschrieben worden (Stahl et al., PNAS, 73 (1976), 4045-4049).

Neben dem oben erwähnten Einfluss auf die Kohlenhydratzusammensetzung ist der prozentuale Anteil an funktionell aktiven (Glyko-) Proteinen in der gereinigten Präparation ein wesentlicher, die Qualität des Produktes bestimmender Faktor, der ebenfalls durch Zellkulturmedium, Fermentationsbedingungen und Reinigungsverfahren beeinflusst werden kann.

Als Methode der Wahl zur Bestimmung des Anteils funktionell aktiver, radiomarkierter Moleküle (z.B. MAk mit physiologisch intakter V-Region) wird der von Lindmo et al. (Immunological Methods 72 (1984), 77) zum ersten Mal beschriebene quantitative Bindungsassay im Antigen-Überschuss verwendet. Diese Methode erlaubt es, den Anteil immunreaktiver und bindefähiger radiomarkierter MAk nach proteinchemischer Reinigung quantitativ zu bestimmen. Andere Bindungsassays, wie der von Seitz et al. (European Journal of Nuclear Medicine 26 (1999), 1265) beschriebene Immunreaktivitätstest, bei dem Immunreaktivitäten von bis zu 100 % beschrieben sind, erlauben nur eine relative Bestimmung des immunreaktiven Anteils von radiomarkierten MAk im Vergleich zum unmarkierten Standard und sind somit zur Bestimmung der absoluten immunreaktiven Fraktion nicht geeignet. Auch das von Jagoda et al. (Journal of Immunological Methods 173 (1994), 191-201) beschriebene affinitäts-chromatographische Verfahren zur Bestimmung der Immunreaktivität ist aufgrund des relativ hohen Anteils an unspezifischer Bindung an die Affinitätssäule (10-20 %) für eine quantitative Bestimmung des immunreaktiven Anteils nur bedingt verwendbar.

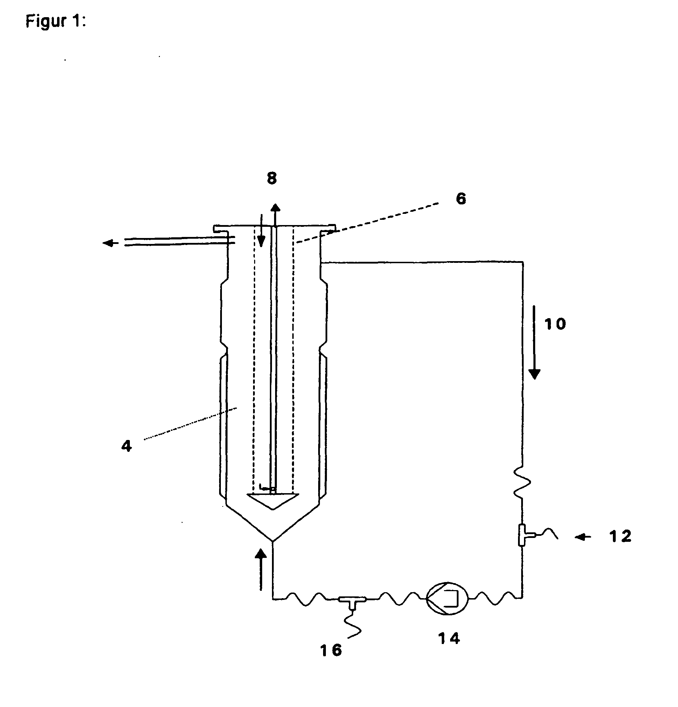

Aber auch der von den meisten Autoren verwendete Lindmo-Test ist trotz des Anspruchs der Autoren auf Generalisierbarkeit der Methode nur zur Evaluierung von radiomarkierten MAk geeignet, deren Bindung unter Verwendung steigender Antigenmengen ein distinktes Plateau ergeben. Mit MAk niedriger Avidität oder im Falle des Mangels an Zellen, die hohe Antigendichten pro Zelle exprimieren, wird oft kein distinktes Plateau erreicht (Mattes, M.J., Int. J. Cancer: 61 (1995), 286-288). Selbst in Untersuchungen, bei denen ein definiertes Plateau erreicht wird, können nach Auftragen der Werte aus den Sättigungskurven entsprechend der von Lindmo et al. vorgegebenen Methode zwei sich schneidende Geraden entstehen, deren Schnittpunkt mit der Y-Achse zu unterschiedlichen Immunreaktivitäten führen kann (Mattes, M.J., Int. J. Cancer: 61 (1995), 286-288, Seite 287, Figur 1 B und E). Bei Verwendung der Lindmo-Methode muss deshalb zwangsläufig sowohl der maximale spezifisch gebundene radioaktive Anteil und der bis zum unendlichen Antigen-Überschuss extrapolierte Wert angegeben werden. Wenn die Differenz zwischen diesen beiden Werten > 10 % ist, kann der extrapolierte Immunreaktivitätswert nicht als verlässlich angesehen werden (Mattes, M.J., Int. J. Cancer: 61 (1995), 286-288, Seite 288).

Unter Berücksichtigung der oben aufgezeigten methodischen Bedenken bewegt sich der prozentuale Anteil funktionell aktiver MAk-Moleküle in mit konventionellen Fermentationsverfahren, wie gerührte Fermenter und Hohlfasermodule, hergestellten und mit klassischen proteinchemischen Verfahren, die neben Protein A-Säulen auch Ionenaustauschersäulen und in vielen Fällen auch gelchromatographische Säulen enthalten, gereinigten Präparationen im Bereich von 40 bis 80 %, wenn der Lindmo-Test verwendet wird (Mattes, M.J., Int. J. Cancer: 61 (1995), 286-288; Jagoda et al., Journal of Immunological Methods 173 (1994), 191-201; Morales-Morales et al., Nuclear Medicine & Biology, Vol. 26 (1999), 275-279; Boven et al., Blood, 67, No. 2 (1986), 429-435). Hierbei spielt das verwendete Radiomarkierungsverfahren und das verwendete Radioisotop ebenfalls eine wichtige Rolle.

Dies bedeutet, dass herstellungsbedingt 20 bis 60 % der MAk-Moleküle dieser Präparationen nicht die erwünschte diagnostische oder therapeutische Funktion vermitteln.

Bruynck et al. (Br. J. Cancer 67 (1993), 436-440) beschreiben die Charakterisierung eines humanisierten bispezifischen monoklonalen Antikörpers für die Tumortherapie mit einem für das karzinoembryonale Antigen (CEA) spezifischen Arm und einem gegen das radiomarkierte Chelat DTPA-$^{90}$Y gerichteten Arm. Die Antikörper wurden durch eine Doppel-Immunaffinitätschromatographieprozedur gereinigt. Es wird eine Immunreaktivität von 95 % für den nicht radioaktiv markierten bispezifischen MAk beschrieben.

WO86/05202 beschreibt einen Wirbelschicht-Bioreaktor und dessen Verwendung zur Kultivierung von Zellen, u.a. von Hybridomzellen zur Gewinnung monoklonaler Antikörper. Es findet sich keinerlei Hinweis, dass durch das Verfahren Antikörper mit hoher Immunreaktivität erhalten werden können.

EP-A-0 727 480 beschreibt ein Verfahren zur Bestimmung von Pharmakokinetik bzw. Toxikokinetik von Testsubstanzen mit Hilfe von *in vitro* Zellkultursystemen und als dafür geeignete Vorrichtung einen Wirbelschicht-Bioreaktor. Es findet sich keinerlei Hinweis auf die Verwendung dieses Wirbelschicht-Reaktors zur Gewinnung von monoklonalen Antikörpern.

WO 96/22310 beschreibt die Markierung eines Antikörpers mit einem Radionuklid ($^{125}$I).

Die Fraktion der immunreaktiven markierten Antikörper wird mittels Standard-Lindmotest ermittelt und wird als 99% angegeben.

Wie die beschriebenen Antikörper aufgereinigt wurden ist nicht offenbart.

Waibel et al. (Nature Biotechnology 17(1999), 897-901) beschreiben Präparationen rekombinanter Einzelkettenantikörper, deren Anteil an immunreaktiven Moleküle mittels Standard-Lindmotest ermittelt und als 57-97% angegeben wird.

Eine literaturbekannte Ausnahme stellt das von Schubiger et al. (Eur. J. Nucl. Med. 15: (1989), 605-608) beschriebene Granuloszint dar, bei dem eine Immunreaktivität von 93 bzw. 40 %, abhängig vom jeweiligen Markierungsverfahren, beschrieben wird. Allerdings wurde dieser MAk in Form von Aszites (persönliche Mitteilung) hergestellt. Dieses in vivo Verfahren erfüllt weder ökonomische noch moderne regulatorische Bedingungen und muss hier als Ausnahme betrachtet werden. Desweiteren muss hier bemerkt werden, dass der Anteil der wirklich spezifisch bindenden MAk-Moleküle geringer ist als der im Lindmo-Test bestimmte Wert. Dies ist bedingt durch die unspezifische Bindung von radiomarkierten MAk an z.B. im Antigen-Überschuss verwendeten Granulozyten trotz Zugabe eines 10.000fachen molaren Überschusses an unmarkiertem spezifischem MAk (Schubiger et al., Euro J. Nucl. Med., 15 (1989), 605-608). Demzufolge liegt der Immunreaktivitätswert nach Subtraktion der unspezifischen Bindung bei <90 %.

Im Falle von radioaktiv markierten diagnostischen monoklonalen Antikörpern ist eine verringerte Immunreaktivität des MAk von besonderem Nachteil, da radiomarkierte, nicht an die spezifische Zielstruktur bindende Moleküle im Blut zirkulieren, zum unspezifischen Hintergrund beitragen, teilweise in die Leber aufgenommen werden und die Interpretation von nuklearmedizinischen Befunden erschweren. Im Falle von radiomarkierten therapeutischen Antikörpern trägt der Anteil nicht an das Zielgewebe gebundener MAk-Moleküle zur unspezifischen und unerwünschten Bestrahlung von Nicht-Zielgeweben bei.

Daher ist es vor allem für die Herstellung von radiomarkierten monoklonalen Antikörpern von größter Bedeutung, ökonomisch und regulatorisch akzeptable in vitro Verfahren zu entwickeln, die eine Herstellung von MAk-Präparationen mit möglichst hohem immunreaktivem Anteil erlauben.

Zur Zeit ist es nur durch den Einsatz aufwendiger und für eine nachfolgende Pharmaherstellung nicht geeigneter, immunaffinitätschromatographischer Verfahren möglich, einen sehr hohen immunreaktiven Anteil zu erreichen. Hierbei werden die funktionell aktiven Moleküle von den funktionell inaktiven Molekülen, die sich proteinchemisch von den aktiven Molekülen nicht unterscheiden, durch Bindung an Antigensäulen getrennt und dadurch auch aufkonzentriert. Trotz des hohen Aufwandes bei der Herstellung der für die Immunaffinitätschromatographie benötigten Reagenzien sowie der hohen Verluste bei der Reinigung, ist der Anteil an funktionell aktiven MAk in solchen, für Forschungszwecke verwendeten Präparationen <81 %.

Um sowohl den immunreaktiven Anteil von nicht markierten MAk als auch von radiomarkierten MAk unbeeinflusst durch unspezifische Bindungen sicher messen zu können, wurde ein "modifizierter Lindmo-Test" entwickelt, der in Beispiel 3 detailliert beschrieben ist. In diesem modifizierten Lindmo-Test beeinflusst der Anteil unspezifisch bindender MAk-Moleküle (wie z.B. nach Radiomarkierung im Beisein eines 10.000fachen Überschusses an kaltem MAk gefunden, Schubiger et al., Euro J. Nucl. Med., 15 (1989), 605-608) den berechneten Immunreaktivitätswert nicht.

Im Rahmen der Arbeiten zur Optimierung der Herstellung des Produktionsverfahrens für den monoklonalen Antikörper BW 250/183 (Eur. J. Nucl. Med. 14 (1988), 523-528 und Int. J. Cancer 36 (1985), 75-84) ist es überraschenderweise gelungen, ein Fermentationsverfahren aufzubauen, welches Zellkulturüberstände erzeugt, die MAk enthalten, deren Immunreaktivität im modifizierten Lindmo-Test in der Regel >95 % beträgt.

Aus diesen Zellkulturüberständen können unter Verwendung von konventionellen ReinigungsverfahrenMAk-Präparationenhergestelltwerden (auch GMP-gerecht), die eine Immunreaktivität zwischen 80 und 90 % haben. Der hier zu beobachtende Verlust an Immunreaktivität von ca. 10 % im Verlauf der Reinigung ist durch die Vielzahl von säulenchromatographischen Schritten bedingt.

Weiterhin ist es gelungen, ein "säulenarmes" Reinigungsverfahren zu entwickeln, welches es erlaubt, MAk-Präparationen herzustellen (auch GMP-gerecht), die im modifizierten Lindmo-Test eine Immunreaktivität haben, die sich von der der ungereinigten MAk-Moleküle im Zellkulturüberstand nicht signifikant unterscheidet (Verlust von ca. 1-2 %).

Diese Verfahren lassen sich auf eine Vielzahl von MAk unterschiedlicher Spezifität und proteinchemischer Zusammensetzungen sowie auf andere immunreaktive Proteine anwenden und führen auch in diesen Fällen zu vergleichbar überraschenden Ergebnissen.

Die Erfindung betrifft somit eine Präparation von immunreaktiven Proteinen, wobei der in einem modifizierten Lindmo-Test gemäß Beispiel 3 bestimmte prozentuale Anteil an immunreaktiven Molekülen >90 % und vorzugsweise > 95 % bezüglich der Gesamtzahl von Molekülen ist wobei es sich bei dem immunreaktiven Protein um einen monoklonalen, chimärisierten oder humanisierten Antikörper handelt, der an ein Epitop auf CD66 bindet. Die immunreaktiven Proteine sind durch Herstellung in Zellkultur, insbesondere in eukaryontischen Wirtszellen, erhältlich. Die immunreaktiven Proteine können ohne Aktivitätsverlust mit einer Markierung, insbesondere mit einer radioaktiven Markierung, gekoppelt werden.

Immunreaktive Proteine im Sinne der vorliegenden Erfindung sind monoklonale Antikörper, chimärisierte Antikörper, humanisierte Antikörper.

Die erfindungsgemäßen Proteinpräparationen sind durch Fermentation in einem Wirbelschichtreaktor erhältlich. In einem solchen Wirbelschichtreaktor wachsen die kultivierten Wirtszellen, z. B. Hybridomzellen oder andere eukaryotische Zellen, in sehr hoher Dichte auf Glaskugeln und können optimal mit Sauerstoff und Nährstoffen versorgt werden. Absterbende Zellen lösen sich von den Glaskugeln ab und werden kontinuierlich mit dem geernteten Medium aus dem Fermentationssystem entfernt. Hierbei können die Mengen von Proteasen und Zelldebris im Fermentationssystem gering gehalten und die Integrität des erzeugten Proteins gewährleistet werden. Ein Beispiel für einen geeigneten Wirbelschicht-Fermentationsreaktor ist der Bioreaktor Pilot B 500 (Papaspyrou Biotechnologie GmbH).

Die Messung der Immunreaktivität der erfindungsgemäßen Proteinpräparationen erfolgt durch den in Beispiel 3 beschriebenen modifizierten Lindmo-Test. Bei der Durchführung dieses Tests ist wesentlich, dass das Antigen-enthaltende Material im Überschuss eingesetzt wird, um das immunreaktive Protein, z.B. den MAk, quantitativ zu binden. Desweiteren ist wichtig, dass das Antigen-enthaltende Material eine ausreichende Menge an Epitopen enthält, damit unspezifische, durch große Oberflächen bedingte Adsorptionseffekte vernachlässigt werden können. So sind Epitopdichten von $> 10^4$ pro fixierter Zelle bevorzugt. Der Ablesepunkt für die Immunreaktivität ist dabei derjenige Punkt, bei dem ein Kontroll-MAk (gleicher Isotyp wie der Test-MAk, aber irrelevante Binderegion) keine signifikante unspezifische Adsorption zeigt.

Im Folgenden werden neben dem erfindungsgemäßen Fermentationsverfahren und dem erfindungsgemäßen "säulenarmen" Reinigungsverfahren auch die erhaltenen erfindungsgemäßen Proteinpräparationen sowie deren vorteilhafte Verwendung als Diagnostikum und Therapeutikum beschrieben.

Die beiden erfindungsgemäßen Herstellungsverfahren I und II bestehen je aus zwei Abschnitten, die sich in mehrere Einzelschritte unterteilen lassen:

| Herstellungsverfahren I (allgemein) | | Herstellungsverfahren II (allgemein) | |
|---|---|---|---|
| - | Anzucht von eukaryotischen Zellen und deren Fermentation in einem Wirbelschichtreaktor | - | Anzucht von eukaryontischen Zellen und deren Fermentation in einem Wirbelschichtreaktor |
| - | Proteinchemische Reinigung mit einem konventionellen Reinigungsverfahren | - | Proteinchemische Reinigung mit einem "säulenarmen" Reinigungsverfahren |

**Herstellungsverfahren I (speziell):**

**Anzucht von eukaryontischen Zellen und deren Fermentation in einem Wirbelschichtreaktor**

1. Auftauen von Zellen aus der Zellbank und Kultivierung, z.B. in T-Flaschen
2. Expandieren der Zellen, z.B. in Spinner-Kulturen
3. Fermentation im Wirbelschichtreaktor

**Proteinchemische Reinigung mit einem konventionellen Reinigungsverfahren**

1. Ernte des Zellkulturmediums, Zellabtrennung, z.B. durch Mikrofiltration, Sterilfiltration und Lagerung, z.B. bei -20°C
2. Ankonzentrierung, z.B. durch Ammoniumsulfatfällung und anschließender Zentrifugation
3. Lösung; Virusinaktivierung mittels Detergenz-Behandlung; Sterilfiltration
4. Affinitätschromatographie mit Protein A oder vergleichbarem Affinitätssystem
5. Ankonzentrierung, z.B. durch Ammoniumsulfatfällung und anschließender Zentrifugation
6. Lösung; Umpufferung mittels Gelchromatographie
7. Anionenaustausch-Chromatographie
8. Ankonzentrierung, z.B. durch Ammoniumsulfatfällung und anschließender Zentrifugation
9. Lösung; Umpufferung mittels Gelchromatographie
10. Einstellung der gewünschten Endkonzentration des Bulks
11. Sterilfiltration

**Herstellungsverfahren II (speziell)**

**Anzucht von eukaryontischen Zellen und deren Fermentation in einem Wirbelschichtreaktor**

1. Auftauen von Zellen aus der Zellbank und Kultivierung, z.B. in T-Flaschen
2. Expandieren der Zellen, z.B. in Spinner-Kulturen
3. Fermentation im Wirbelstromreaktor

**Proteinchemische Reinigung mit einem "säulenarmen" Reinigungsverfahren**

1. Ernte des Zellkulturmediums, Zellabtrennung, z.B. durch Mikrofiltration, Sterilfiltration und Lagerung, z.B. bei -20°C
2. Auftauen der sterilen und zellfreien Kultur; Ernte, gefolgt von Mikrofiltration und Ultrafiltration
3. Verdünnung
4. Behandlung mit Detergenz
5. Affinitätschromatographie mit Protein A oder vergleichbarem Affinitätssystem
6. Verdünnung und Sterilfiltration
7. Anionenaustausch-Membranadsorption
8. Virusfiltration mittels Ultrafiltration
9. Ultrafiltration und Diafiltration
10. Filtration und Verdünnung
11. Sterilfiltration

Die oben aufgezeigten individuellen Herstellungsschritte sind als jeweilige Einzelschritte dem Fachmann (Zellbiologen/Proteinchemiker) methodisch bekannt und bedürfen keiner nähreren Beschreibung. Trotzdem ist das Verfahren ausführlich in Beispiel 2 beschrieben.

Neu an Herstellungsverfahren I ist die Fermentation im Wirbelschichtreaktor in Kombination mit einem konventionellen Reinigungsverfahren.

Überraschenderweise beträgt die Immunreaktivität des MAk in ungereinigten Zellkulturüberständen (T-Flaschen, Wirbelschichtreaktor) >90 % im modifizierten Lindmo-Test. Nach konventioneller Reinigung werden Immunreaktiviäten von >80 % erreicht.

Neu an Herstellungsverfahren II ist die Fermentation mittels Wirbelschichtreaktor in Kombination mit dem "säulenarmen" Reinigungsverfahren, in dem nur eine Säulenchromatographie (Affinitätschromatographie an Protein A) verwendet wird. Alle sonstigen Schritte stellen schonende Filtrationsschritte durch Membranen oder einfache Verdünnungsschritte dar. Trotzdem werden mit dem Verfahren die von den Zulassungsbehörden festgelegten Forderungen bezüglich proteinchemischer Reinheit und Virusabreicherung/-inaktivierung erfüllt.

Überraschenderweise beträgt die Immunreaktivität des herzustellenden (Glyko-) Proteins, z.B. eines MAk, auf allen getesteten Stufen (T-Flaschen, Wirbeschichtreaktor, gereinigtes Protein) >90 % im modifizierten Lindmo-Test gemäß Beispiel 3 meist sogar 95 %, wie beispielhaft in Beispiel 1 für den MAk BW 250/183 gezeigt. Vergleichbar hohe Immunreaktivitätswerte sind unseres Wissens mit keinem in dem Stand der Technik beschriebenen in vitro Herstellungsverfahren erreicht worden.

Ähnlich hohe Immunreaktivitätswerte wurden mit dem erfindungsgemäßen Verfahren für andere MAk, wie z.B. den MAk BW 431/26 (selektiv für CD66e) (Eur. J. Nucl. Med. 14 (1988), 523-528 und Int. J. Cancer 36 (1985), 75-84), MAk BW 278/105 (selektiv für eine Subpopulation von FVIII RAG) (J. Histochem. Cytochem. 34 (1986), 209-214), MAk BW 575/931 (selektiv für N-CAM) (Pediatr. Hematol. Oncol. 6 (1989), 73-83) und MAk YTH 24.5 (selektiv für CD45) (J. Immunol. 134 (1985), 3056-3061 und Leucocyte Typing III: White Cell Differentiation Antigens (Hrsg. Mc Michael et al.), Oxford University Press, Oxford, pp 788-803) sowie einige der in der Deutschen Patentanmeldung 197 44 531.4 beschriebenen MAk (selektiv für den VEGF/VEGF-Rezeptor-Komplex, binden aber weder an VEGF noch an VEGF-Rezeptor alleine) gefunden. Diese Befunde belegen, dass die erfindungsgemäßen Herstellungsverfahren es erlauben, (Glyko-) Proteine zu fermentieren, die zu annähernd 100 % immunreaktiv sind, und diese schonend und schnell, im Falle von Herstellungsverfahren II sogar ohne nachweisbare Verluste an Immunreaktivität kostengünstig zu reinigen.

Desweiteren sind die Herstellungsverfahren für die GMP-gerechte Herstellung einer Vielzahl von (Glyko-) Proteinen, die keine Fc-Teile haben, einsetzbar, unter anderem für das von Bosslet et al. beschriebene Fusionsprotein (British Journal of Cancer, 645 (1992), 234-238), wobei anstelle der Protein A-Affinitätschromatographie ein alternatives affinitätschromatographisches Verfahren eingesetzt wird (anti-Idiotyp, Lektinsäule, Protein L, Nickelsäure etc.)

Neben der Immunreaktivität werden auf den jeweiligen Herstellungsstufen eine Vielzahl von Qualitätskontrollen (Sterilität, DNA-Gehalt, Protein-Gehalt, Spezifität, pH-Wert, Proteinzusammensetzung, Isoelektrischer Punkt, Pyrogene, Protein A-Gehalt im Endprodukt) durchgeführt, welche es erlauben, die mikrobiologischen, immunologischen und proteinchemischen Eigenschaften des herzustellenden (Glyko-) Proteins (MAk) zu testen. Auf diese Tests wird hier nicht eingegangen, da sie Stand der Technik sind.

Weiterhin soll die Erfindung durch die nachfolgenden Beispiele in Verbindung mit den Figuren erläutert werden. Es zeigen:

**Figur 1**

die Beschreibung eines Wirbelschicht-Fermentationsreaktors

Der Reaktor (2) ist mit Trägerkugeln (4), z.B. Kugeln aus offenporigem gesinterten Glas mit einem Durchmesser von vorzugsweise 0,4 bis 0,7 mm, die einer Säurebehandlung (z.B. 2,5 N HCl und 5 % Salpetersäure) unterzogen und nach Neutralisierung durch Hitzebehandlung (z.B. 220 °C, 6 h) sterilisiert wurden, teilweise gefüllt. Der Reaktor enthält weiterhin ein Belüftungsmodul (6), mit dem eine Gasmischung (8) zur Verwirbelung der Trägerkugeln in den Reaktor eingeblasen und wieder herausgeleitet wird. Weiterhin ist der Reaktor mit pH-Elektroden, Sauerstoffelektroden, Temperatursonden etc. ausgestattet. Das Medium wird mit einer Fließgeschwindigkeit von beispielsweise 450 bis 500 ml/min in einem Kreislauf (10) rezirkuliert. Der Kreislauf kann einen Einlass frisches Medium (12), eine Pumpe (14) und ein Probenentnahmeventil (16) enthalten. Durch die Leitung wird Medium zur Ernte des darin enthaltenen Proteins entnommen.

**Figur 2**

Ergebniskurven für Antikörperpräparationen in einem modifizierten Lindmo-Test

**Figur 3 und 4**

Szintigramme mit TC-99m-markierten monoklonalen Antikörpern aus Präparationen des Standes der Technik.

**Figur 5 bis 7**

Szintigramme von TC-99m-markierten monoklonalen Antikörpern aus erfindungsgemäßen Präparationen.

Im Folgenden sind die Ergebnisse einer typischen Immunreaktivitätsbestimmung für einen MAk, im Beispiel für den MAk BW 250/183, aufgeführt.

**Beispiel 1**

Verglichen wird die Immunreaktivität des MAk BW 250/183 (Eur. J. Nucl. Med. 14 (1988), 523-528), gemessen mit dem modifizierten Lindmo-Test (Durchführung siehe Beispiel 3), bei konventioneller Fermentation und Reinigung gegenüber den beiden neuen, hier beschriebenen Fermentations- und Reinigungsverfahren (Herstellverfahren I und Herstellverfahren II) jeweils vor Beginn der Reinigung (Zellkulturüberstand) und nach Ende der Reinigung (gereinigtes MAk-Endprodukt).

Die MAk-haltigen Proben der Zellkulturüberstände bzw. der gereinigten MAk-Endprodukte wurden, wie in Beispiel 3 genau beschrieben, behandelt und der modifizierte Lindmo-Test laut Anweisung durchgeführt. Als Negativ-Kontrolle wurde ein MAk vom gleichen Isotyp (IgG$_1$), gleicher leichter Kette ($k$), vergleichbarem isoelektrischem Punkt und irrelevanter Spezifität eingesetzt. Die Positivkontrolle ist ein über eine Antigensäule im analytischen Maßstab gereinigte Charge des MAk BW 250/183 mit einer Immunreaktivität von 91 bis 94 %.

Im Folgenden sind die Ergebnisse der einzelnen Immunreaktivitätsbestimmungen der Zellkulturüberstände sowie der gereinigten MAk-Endprodukte aufgeführt:

**1. Konventionelles Fermentationsverfahren und konventionelles Reinigungsverfahren (Stand der Technik)**

a) Nichtgereinigter Zellkulturüberstand (ZKÜ) nach koventioneller Fermentation (KF)

Tabelle 1:

|  | nachgewiesene Masse in ng | Ausgangsmasse in ng | Immunreaktivität |
|---|---|---|---|
| Negativkontrolle | - | 50,0 | - |
| Positivkontrolle | 3,2 | 48,0 | 93,3 % |
| 250/183, ZKÜ, KF, Probe 1 | 7,5 | 40,7 | 81,6 % |
| 250/183, ZKÜ, KF, Probe 2 | 8,2 | 38,6 | 78,8 % |
| 250/183, ZKÜ, KF, Probe 3 | 8,5 | 40,2 | <u>78,9 %</u> |
| IR-Mittelwert der 3 Proben: |  |  | **79,7** % |

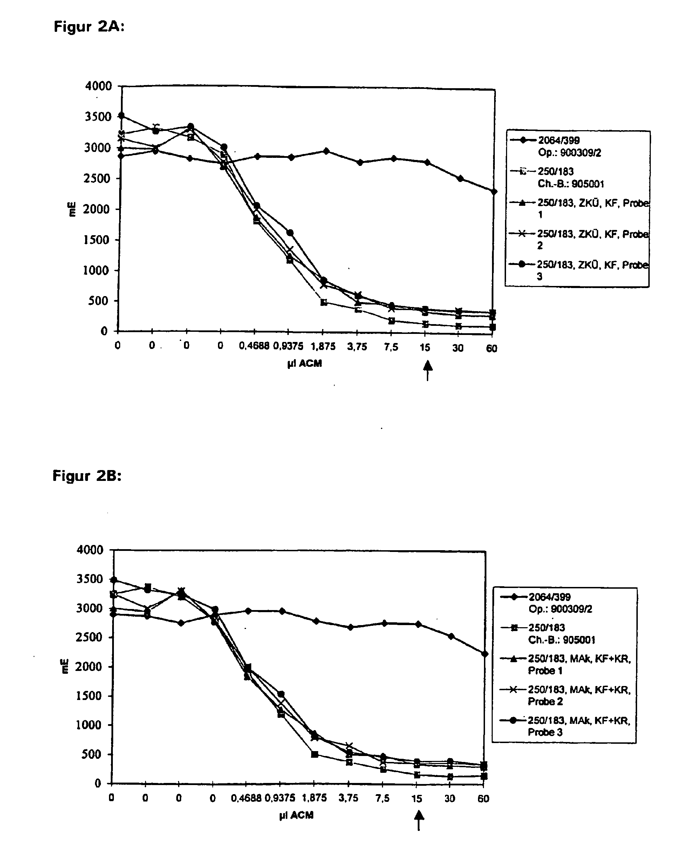

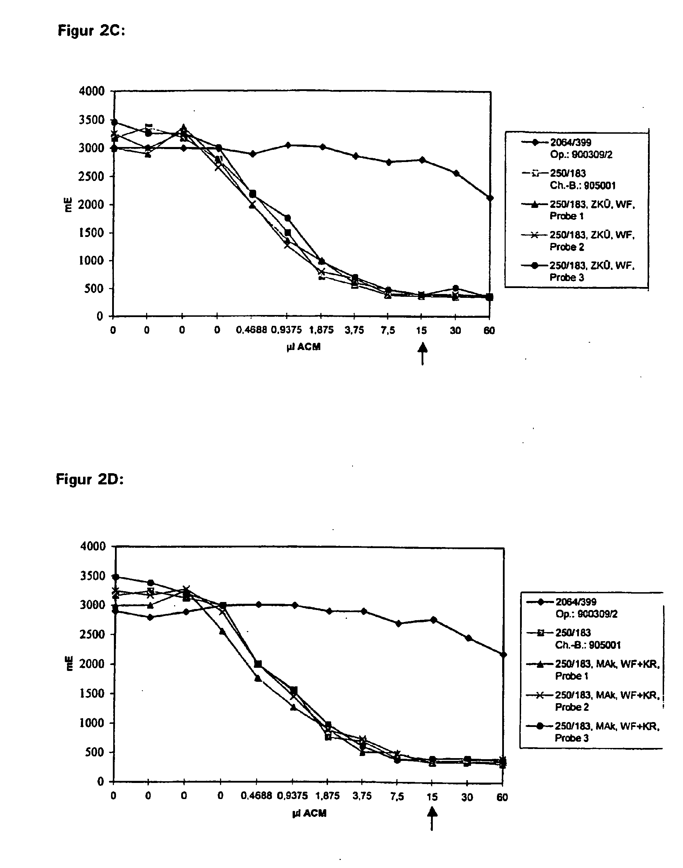

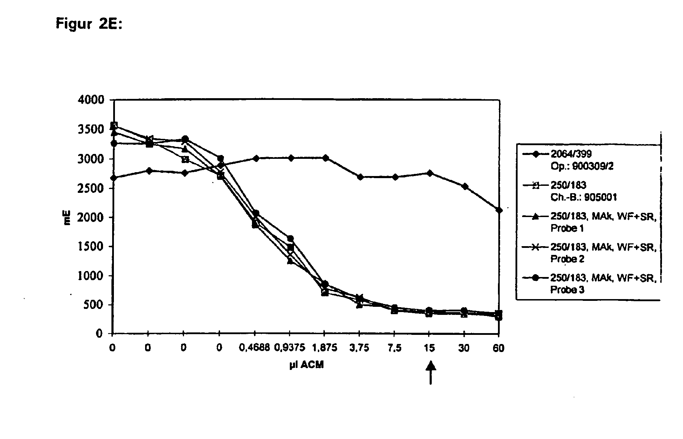

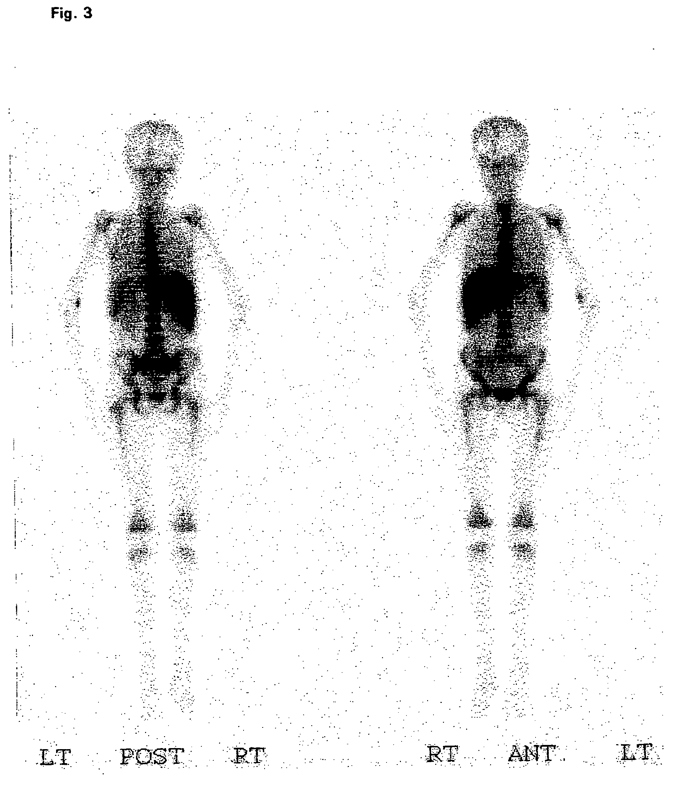

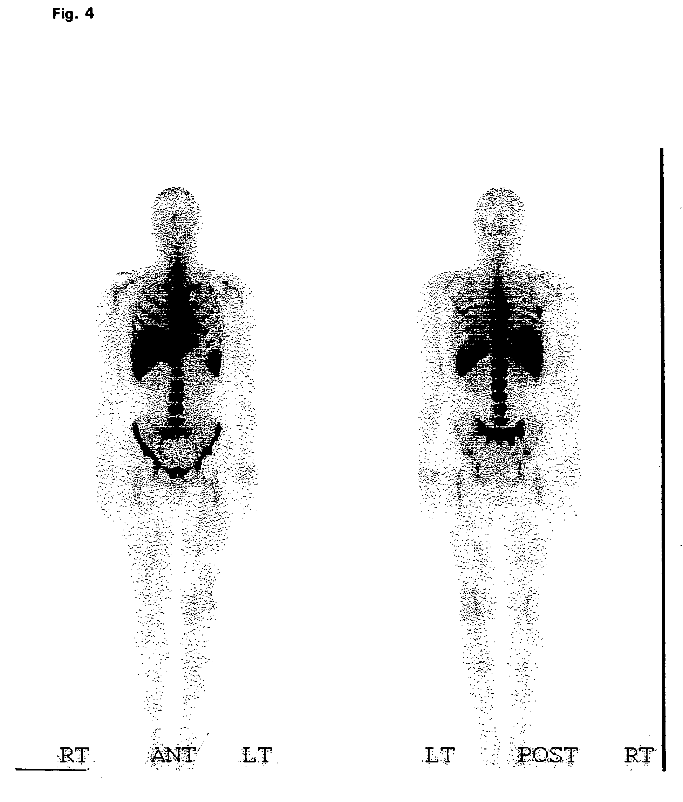

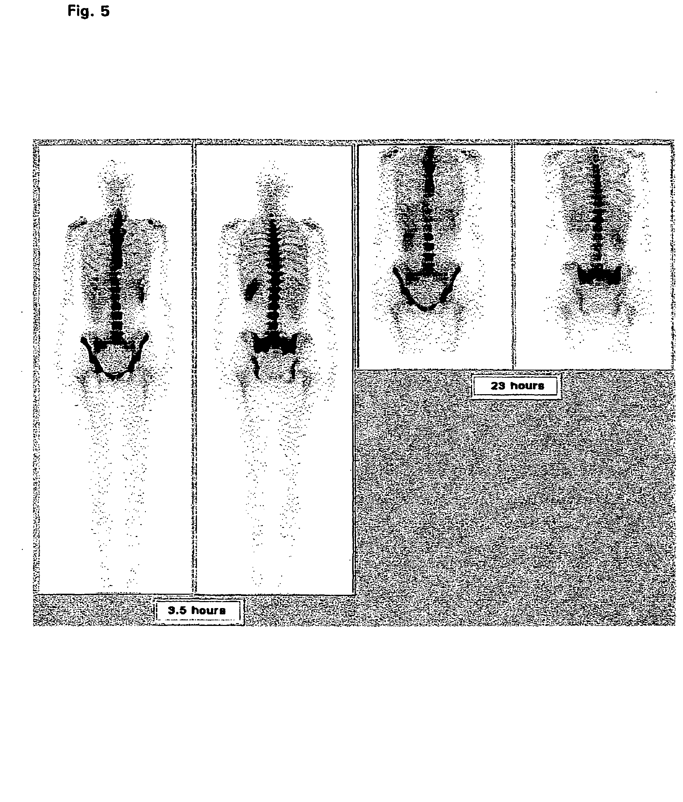

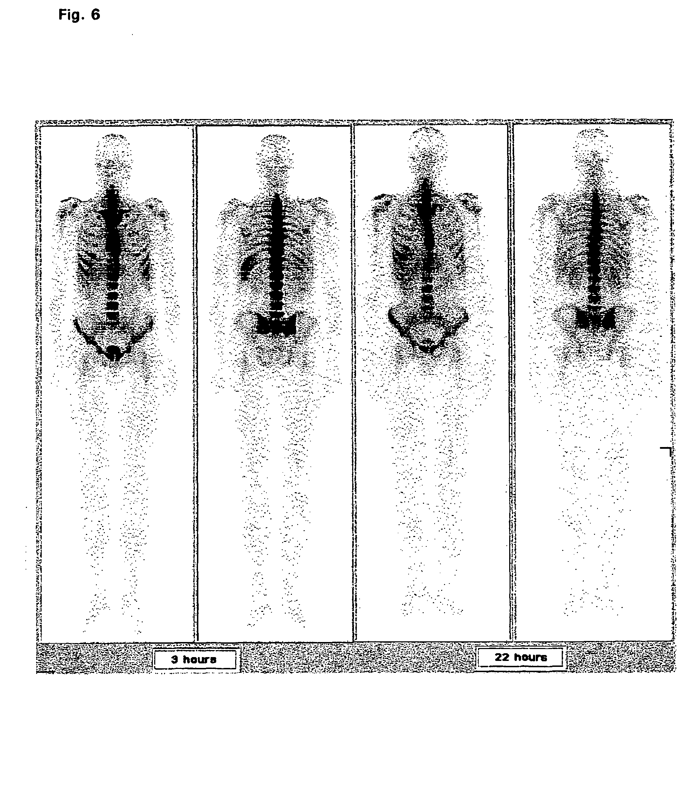

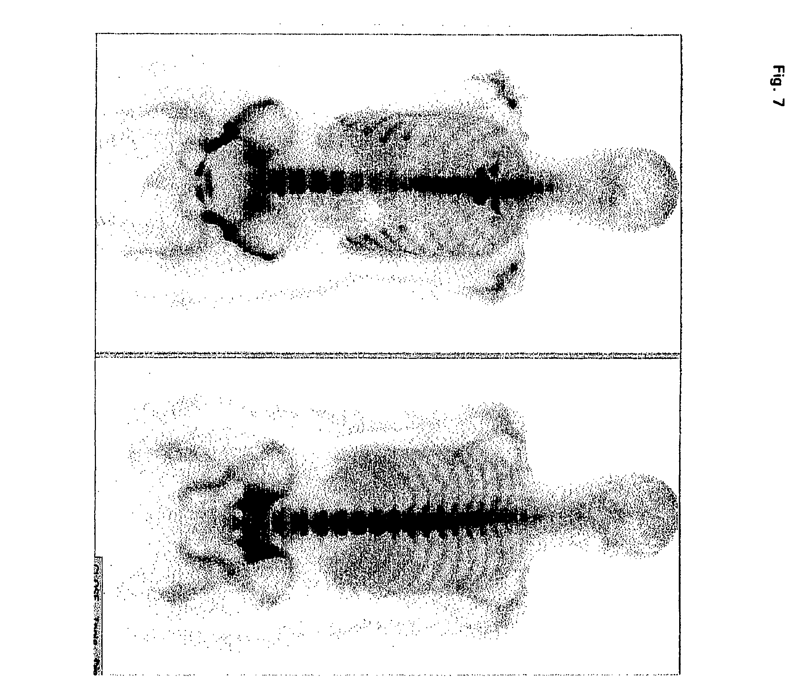

Die Ergebniskurve ist in Figur 2A dargestellt.

b) Gereinigtes MAk-Endprodukt (MAk) nach konventioneller Fermentation (KF) und konventioneller Reinigung (KR)

Tabelle 2:

|  | nachgewiesene Masse in ng | Ausgangsmasse in ng | Immunreaktivität |
|---|---|---|---|
| Negativkontrolle | - | 50,0 | - |
| Positivkontrolle | 3,2 | 48,0 | 93,3 % |
| 250/183, MAk, KF + KR, Probe 1 | 7,3 | 24,8 | 70,6 % |
| 250/183, MAk, KF + KR, Probe 2 | 8,1 | 28,8 | 71,9 % |
| 250/183, MAk, KF + KR, Probe 3 | 8,4 | 32,4 | <u>74,1 %</u> |
| IR-Mittelwert der 3 Proben: |  |  | **72.2** % |

Die Ergebniskurve ist in Figur 2B dargestellt.

**2. Fermentation im Wirbelschichtreaktor und konventionelles Reinigungsverfahren oder "säulenarmes" Reinigungsverfahren (Erfindung)**

a) Zellkulturüberstand (ZKÜ) nach Wirbelschicht-Fermentation (WF)

Tabelle 3:

|  | nachgewiesene Masse in ng | Ausgangsmasse in ng | Immunreaktivität |
|---|---|---|---|
| Negativkontrolle | - | 50,0 | - |
| Positivkontrolle | 3,5 | 45,2 | 92,4 % |
| 250/183, ZKÜ, WF, Probe 1 | 3,7 | 183,5 | 98,0 % |

(fortgesetzt)

| | nachgewiesene Masse in ng | Ausgangsmasse in ng | Immunreaktivität |
|---|---|---|---|
| 250/183, ZKÜ, WF, Probe 2 | 4,0 | 137,8 | 97,1 % |
| 250/183, ZKÜ, WF, Probe 3 | 3,9 | 123,0 | 96,8 % |
| IR-Mittelwert der 3 Proben: | | | **97,3 %** |

Die Ergebniskurve ist in Figur 2C dargestellt.

b) Gereinigtes MAk-Endprodukt (MAk) nach Wirbelschicht-Fermentation (WF) und konventioneller Reinigung (KR)

**Tabelle 4:**

| | nachgewiesene Masse in ng | Ausgangsmasse in ng | Immunreaktivität |
|---|---|---|---|
| Negativkontrolle | - | 50,0 | - |
| Positivkontrolle | 3,5 | 45,2 | 92,4 % |
| 250/183, MAk, WF + KR, Probe 1 | 3,6 | 39,5 | 90,9 % |
| 250/183, MAk, WF+KR, Probe 2 | 4,1 | 35,6 | 88,5 % |
| 250/183, MAk, W + KR, Probe 3 | 3,9 | 34,6 | 88,7 % |
| IR-Mittelwert der 3 Proben: | | | **89,3 %** |

Die Ergebniskurve ist in Figur 2D dargestellt.

c) Gereinigtes MAk-Endprodukt (MAk) nach Wirbelschicht-Fermentation (WF) und "säulenarmer" Reinigung (SR)

**Tabelle 5:**

| | nachgewiesene Masse in ng | Ausgangsmasse in ng | Immunreaktivität |
|---|---|---|---|
| Negativkontrolle | - | 50,0 | - |
| Positivkontrolle | 3,5 | 45,2 | 92,4 % |
| 250/183, MAk, WF+SR, Probe 1 | 3,5 | 81,4 | 95,7 % |
| 250/183, MAk, WF + SR, Probe 2 | 3,9 | 99,5 | 96,1 % |
| 250/183, MAk, W + SR, Probe 3 | 4,0 | 100,5 | 96,0 % |
| IR-Mittelwert der 3 Proben: | | | **95,9 %** |

Die Ergebniskurve ist in Figur 2E dargestellt.
Die Endergebnisse des Beispiels 1 sind in folgender **Tabelle 6** zusammengefasst:

**Immunreaktivität des MAk BW 250/183**

| | konventionelles Fermentations- und konventionelles Reinigungsverfahren | **Herstellverfahren 1** Wirbelschichtreaktor-Fermentation und konventionelles Reinigungsverfahren | **Herstellverfahren 2** Wirbelschichtreaktor-Fermentation und "säulenarmes" Reinigungsverfahren |
|---|---|---|---|
| Zellkulturüberstand | 80 % | 97 % | 97 % |

(fortgesetzt)

| | konventionelles Fermentations- und konventionelles Reinigungsverfahren | Herstellverfahren 1 Wirbelschichtreaktor-Fermentation und konventionelles Reinigungsverfahren | Herstellverfahren 2 Wirbelschichtreaktor-Fermentation und "säulenarmes" Reinigungsverfahren |
|---|---|---|---|
| gereinigtes MAk-Endprodukt | 72 % | 89 % | 96 % |

Die Immunreaktivität des MAk BW 250/183 ist deutlich von den Fermentations- und Reinigungsbedingungen abhängig. Bei konventioneller Fermentation werden zwar immerhin schon typischerweise bis zu 80 % Immunreaktivität im Zellüberstand erreicht - im neuen Fermentationsverfahren mittels Wirbelschichtreaktor aber ist die Immunreaktivität mit 97 % deutlich höher.

Auch das Reinigungsverfahren beeinflusst die Immunreaktivität von MAk. Das neue "säulenarme" Reinigungsverfahren ist deutlich schonender als das konventionelle Reinigungsverfahren (8 % Abfall der Immunreaktivität beim konventionellen Verfahren gegenüber nur 1 % Abfall beim neuen "säulenarmen" Reinigungsverfahren).

Vergleichbare Ergebnisse konnten ebenfalls mit anderen MAk, z.B. mit MAk BW 431/26 (Eur. J. Nucl. Med. 14 (1988), 523-528 und Int. J. Cancer 36 (1985), 75-84), MAk BW 575/931 (Pediatr. Hematol. Oncol. 6 (1989), 73-83), MAk YTH 24.5 (J. Immunol. 134 (1985), 3056-3061 und Leucocyte Typing III: White Cell Differentiation Antigens (Hrsg. Mc Michael et al.), Oxford University Press, Oxford, pp 788-803), MAk BW 278/105 (J. Histochem. Cytochem. 34 (1986), 209-214), den in der Patentanmeldung 197 44 531.4 beschriebenen MAk sowie dem British J. Cancer 645, 234-238, 1992, beschriebenen Fusionsprotein erzielt werden (siehe Tabelle 7).

**Tabelle 7:**

| Immunreaktivität in Abhängigkeit vom Fermentations- und Reinigungsverfahren | | | |
|---|---|---|---|
| Bezeichnung des MAk | konventionelles Fermentations- und konventionelles Reinigungsverfahren | Herstellverfahren 1 Wirbelschichtreaktor-Fermentation und konventionelles Reinigungsverfahren | Herstellverfahren 2 Wirbelschichtreaktor-Fermentation und "säulenarmes" Reinigungsverfahren |
| BW 431/26, ZKÜ* | 85 % | 96 % | 96 % |
| BW 431/26, GME* | 79 % | 87 % | 94 % |
| BW 575/931, ZKÜ* | 75 % | 93 % | 93 % |
| BW 575/931, GME* | 67 % | 85 % | 91 % |
| YTH 24.5, ZKÜ* | 83 % | 98 % | 98 % |
| YTH 24.5, GME* | 77 % | 89 % | 96 % |
| BW 278/105, ZKÜ* | 87 % | 94 % | 94 % |
| BW 278/105, GME* | 81 % | 85 % | 92 % |
| Fusionsprotein ZKU* | 84 % | 97 % | 97 % |
| Fusionsprotein GME* | 76 % | 88 % | 96 % |
| ZKÜ: Zellkulturüberstand GME: gereinigtes MAk-Produkt | | | |

Da vergleichbare Befunde mit allen getesteten MAk und einem sehr komplexen (Glyko-) Protein, dem Fusionsprotein (Molekulargewicht des Tetramers unter nativen Bedingungen 500 kDA) erzielt wurden, darf davon ausgegangen werden, dass vergleichbare Werte mit allen in prokaryontischen und eukaryontischen Systemen fermentierbaren MAk und (Glyko-) Proteinen erhalten werden und die vorteilhaften Befunde generalisierbar sind.

Sowohl die nach dem konventionellen Herstellverfahren als auch den Herstellverfahren I und II gereinigten MAk-Chargen zeigten nach Radiomarkierung entsprechend der von Schwarz und Steinstraesser geschriebenen Methode (J. Nucl. Med., 28 (1987), 721) eine Immunreaktivität, die mit der des gereinigten MAk-Proteins im Rahmen der Genauigkeit des modifizierten Lindmo-Tests identisch war. Die Daten sind in der nachstehenden **Tabelle 8** zusammengefasst:

| gereinigtes MAk-Endprodukt | konventionelles Fermentations- und konventionelles Reinigungsverfahren | **Herstellverfahren 1** Wirbelschichtreaktor-Fermentation und konventionelles Reinigungsverfahren | **Herstellverfahren 2** Wirbelschichtreaktor-Fermentation und "säulenarmes" Reinigungsverfahren |
|---|---|---|---|
| unmarkiert | 72 % | 89 % | 96 % |
| Tc-99m markiert | 70 % | 89 % | 95 % |

**Unerwartete klinische Befunde**

Aliquots von Präparationen des MAk BW 250/183, welche entweder nach dem konventionellen Herstellungsverfahren (Batchfermentation in gerührten Fermentern gefolgt von konventioneller proteinchemischer Reinigung; Immunreaktivität 72 %) oder dem neuartigen Herstellungsverfahren II (Wirbelschichtreaktor gefolgt von "säulenarmem" Reinigungsverfahren; Immunreaktivität 96 %) GMP-gerecht produziert wurden, wurden entsprechend der von Schwarz und Streinstraesser beschriebenen Methode (J. Nucl. Med., 28 (1987), 721) mit Tc-99m markiert. Der Anteil des an den MAk gebundenen Isotops betrug in beiden Präparationen 99,9 %. Von diesen radiochemisch identischen MAk-Präparationen wurden Patienten mit dem Verdacht auf entzündliche Erkrankungen bzw. Knochenmarkmetastasen eine Dosis von 10-20 mCi i.v. verabreicht. Mittels einer γ-Kamera wurden Ganzkörperszintigramme im Zeitraum von 2 bis 25 Stunden nach i.v.-Gabe des radiomarkierten MAk aus frontaler und dorsaler Sicht aufgenommen.

Überraschenderweise stellte sich bei Szintigrammen nach Injektion mit MAk-Chargen, die eine Immunreaktivität von >90 % hatten (Herstellungsverfahren II), vorteilhafterweise präferentiell das Knochenmark scharf kontuiert und die Milz mehr oder weniger deutlich dar (siehe Beispiel 3: Abbildungen GRAN 91, GRAN 81 und GRAN 71). Bei MAk-Chargen, die nach einem konventionellen Fermentations- und Reinigungsverfahren hergestellt wurden (Immunreaktivität 70-80 %), war neben einer Knochenmarkdarstellung ganz deutlich die Leber und die Milz zu erkennen (siehe Beispiel 3: Abbildungen GRAN 11 und GRAN 21). Desweiteren zeigen diese Aufnahmen einen höheren Hintergrund, der sich in einem leichten Schleier und einer gewissen Unschärfe der Szintigramme bemerkbar macht.

Als Konsequenz wurden die Epitop-negativen Normalgewebe von Patienten, die MAk-Chargen mit einer Immunreaktivität >90 % erhielten, wesentlich weniger belastet als von Patienten , die mit MAk-Chargen mit einer Immunreaktivität <80 % behandelt wurden. Diese Beobachtungen an Patienten belegen die Bedeutung der Höhe der Immunreaktivität eines spezifischen MAk gegen Granulozyten für die Bildgebung (Szintigraphie) in der Nuklearmedizin. Aber nicht nur für die Bildgebung, sondern auch für die Therapie mit α- und ß-Strahlern zeigen die MAk-Chargen mit einer Immunreaktivität > 90 % deutliche Vorteile. So ist es z.B. nach Markierung von MAk-Chargen mit einer Immunreaktivität ≥90 % möglich, mittels literaturbekannter Methoden $Re^{188/186}$, $Y^{90}$ oder Astatin anzukoppeln, um eine präferentielle Knochenmarkbestrahlung vorzunehmen (Visser et al., J. Nucl. Med. 34 (1993), 1953-1963; Griffith et al., Cancer Res. 51 (1991), 4594-4602; Denora et al., Anticancer Res. 17 (1997), 1735-1744).

So wurde im Rahmen eines Heilversuches ein Kollektiv von 19 Patienten mit akuter myeloischer Leukämie oder chronisch myeloischer Leukämie behandelt. Hierzu wurden Chargen des MAk BW 250/183 (Immunreaktivität >90 %) mit $Re^{188}$ markiert (spezifische Aktivität; 5-7,5 GBq/mg) und 6,5-12,4 GBq i.v. appliziert.

Dosimetrische Untersuchungen lieferten die in der folgenden Tabelle zusammengefassten Daten.

**Tabelle 9:**

| Dosimetrische Untersuchungen nach Radioimmunotherapie mit Re-188 markiertem MAk BW 250/183 | | | | | | | | | |
|---|---|---|---|---|---|---|---|---|---|
| Geschlecht | Alter | Krankheit | Marker | Datum der KM-Transplantation | Organ-Dosis in Gy | | | | |
| | | | | | KM | Mz | Lb | Nie | Lu* |
| f | 39 | AML | T(8;21) | 2/98 | 12,0 | 7,3 | 3,8 | 5,3 | n.d |
| f | 38 | AML | no | 3/98 | 13,0 | 13,2 | 6,2 | 11,3 | n.d |
| m | 52 | AML | no | 3/98 | 8,0 | 7,0 | 5,0 | 11,0 | n.d |
| m | 45 | AML | no | 4/98 | 13,0 | 12,0 | 4,0 | 11,0 | n.d |
| m | 50 | CML | T(12;13) | 4/98 | 12,8 | 18,6 | n.d | 7,6 | n.d |

(fortgesetzt)

| Dosimetrische Untersuchungen nach Radioimmunotherapie mit Re-188 markiertem MAk BW 250/183 | | | | | | | | | |
|---|---|---|---|---|---|---|---|---|---|
| Geschlecht | Alter | Krankheit | Marker | Datum der KM-Transplantation | Organ-Dosis in Gy | | | | |
| | | | | | KM | Mz | Lb | Nie | Lu* |
| f | 19 | c-ALL | no | 5/98 | 5,9 | 12.3 | 1,8 | 7,1 | n.d |
| m | 44 | c-ALL | Ph + | 6/98 | 15,2 | 12,7 | 3,2 | 5,3 | 0,3 |
| f | 17 | AML | no | 7/98 | 18,4 | 10,3 | 4,1 | 5,0 | n.d |
| m | 50 | AML | T (15;17) | 8/98 | 10,9 | 8,6 | 2,7 | 4,4 | 0,4 |
| f | 32 | AML | Deletion Chrom. 6 | 9/98 | 15,7 | 6,8 | 3,0 | 5,1 | 0,9 |
| m | 56 | B-CLL | no | 9/98 | 15,7 | 4,0 | 2,3 | 4,5 | n.d |
| f | 36 | AML | no | 9/98 | 13,0 | 5,6 | 2,3 | 15,1 | 0,6 |
| m | 45 | c-ALL | Ph+ | 11/98 | 6,5 | 11,5 | 2,7 | 4,5 | n.d |
| f | 19 | AML | T(9;11) | 11/98 | 11,4 | n.d | 7,2 | 10,1 | 1,1 |
| f | 40 | 2. AML (MDS) | no | 12/98 | 13,8 | 18,2 | 5,3 | 10,1 | 0,3 |
| m | 51 | AML | Trisomie 6,8,21 Deletion 11q23 | 12/98 | 14,3 | 6,7 | 5,9 | 8,9 | 0,4 |
| m | 20 | CML | Ph+ | 12/98 | 11,9 | 14,3 | 7,0 | 5,5 | n.d |
| f | 47 | 2. AML Plasmocytoma, MDS | T (16;19) P (11;12) | 12/98 | 16,2 | 7,6 | 3,7 | 5,6 | 0,5 |
| m* | 59 | AML | No | 12/98 | 19,0 | 14,0 | 6,0 | 8,2 | 0,7 |
| *KM: Knochenmark, Mz: Milz. Lb: Leber. Nie: Niere, Lu: Lunge | | | | | | | | | |

Die Daten zeigen, dass es möglich ist, mittels Radioimmuntherapie mit dem Re-188 markierten MAk BW 250/183 (Immunreaktivität >90 %) hohe Strahlendosen auf dem Knochenmark und der Milz von Patienten mit Leukämie zu lokalisieren.

Das Patientenkollektiv bestand aus Personen mit einem Rezidiv-Risiko von 40-50 % innerhalb von 2 Jahren. Überraschenderweise induzierte die Radioimmuntherapie keine signifikanten Nebenwirkungen. Danach folgte eine Standardtherapie bestehend aus 12 Gray Ganzkörperbestrahlung und Cyclophosphamid-Gabe. Alle 19 Patienten konnten in eine komplette Remission gebracht werden, was Hinweise für eine zukünftige Rolle der Radioimmuntherapie mit MAk BW 250/183 bei der Behandlung von Leukämien in Verbindung mit Standardtherapien gibt. Hierbei erscheint es denkbar, dass die Radioimmuntherapie die nebenwirkungsreiche Ganzkörperbestrahlung ersetzen wird.

Ein weiterer Vorteil der Verwendung des MAk BW 250/183 mit einer Immunreaktivität >90 % besteht darin, dass die verabreichte MAk-Menge von 1 mg pro Applikation auf 0,5 mg pro Applikation verringert werden konnte. Diese Reduktion führte zu einer HAMA (human-anti-mouse-antibody)-Frequenz, die unter 2 % lag, und damit nicht signifikant von den Hintergrund-HAMA-Werten von Normalpersonen abweicht.

Weiterhin konnten überraschenderweise auch frühe Metastasen im RES (retikuloendothelialen System) des Knochenmarks mit MAk-Präparaten, die eine Immunreaktivität von $\geq$ 90 % hatten, entdeckt werden, die bei dem Präparat mit ca. 80 % Immunreaktivität aufgrund der Überstrahlung durch Lungen- und Leber-Aktivitätsanreicherung nicht eindeutig nachweisbar waren.

Zusammenfassend darf hier festgehalten werden, dass die überraschenden Vorteile bezüglich Bildqualität, diagnostischer Effizienz, Dosiometrie und therapeutischer Effizienz, die durch die Verwendung von hoch immunreaktiven Chargen des MAk BW 250/183 in Verbindung mit α-, β- oder γ-Strahlern entstehen, weder qualitativ noch quantitativ zu erwarten waren und den Weg zu einer effizienteren Diagnostik entzündlicher Prozesse und von Metastasen sowie zur Therapie von Leukämien und anderen Erkrankungen des hämatopoetischen Systems öffnet.

**Beispiel 2**

**Herstellverfahren I**

**Anzucht von eukaryontischen Zellen und deren Fermentation in einem Wirbelschichtreaktor**

Eine Ampulle aus der Arbeitszellbank, die in flüssigem Stickstoff gelagert ist, wird aufgetaut und die darin befindlichen Zellen werden unter Standard-Zellkulturbedingungen (synthetisches proteinfreies Zellkultur-Medium) in T-Flaschen kultiviert. Nachdem eine Gesamtzellzahl von 6-10 x $10^7$ Zellen erreicht ist, wird der Inhalt von 4 bis 6 T-Flaschen in einem 500 ml - Spinnergefäß unter Standard-Zellkulturbedingungen weiter kultiviert. Nachdem eine Zellzahl von 1,5-2 x $10^8$ Zellen erreicht ist, werden die Zellen in 2 Spinnergefäße mit je 1000 ml Volumen transferiert und weiterkultiviert. Nach Erreichen einer Zellzahl von 1,2-1,5 x $10^9$ Zellen werden die Zellen in einen 900 ml Zellkulturmedium und 200 ml Siran$^R$-Carrier enthaltenden Wirbelschichtreaktor (Bioreaktor Pilot B 500, Papaspyrou Biotechnologie GmbH, Technologiezentrum Jülich, D-52428 Jülich) inokuliert und entsprechend der Handhabungsvorschrift der Fa. Papaspyrou Biotechnologie für 60 Tage bei 36,5 °C kultiviert. Sauerstoffgehalt, pH-Wert, Glukosekonzentration sowie der MAk-Gehalt werden in 1-4tägigen Abständen kontrolliert. Während der Fermentatition wird der Zellkulturüberstand kontinuierlich geerntet und bei 4 °C gelagert.

**Proteinchemische Reinigung mit einem konventionellen Verfahren**

Das Volumen des aufzuarbeitenden Kulturultrakonzentrates wird mit festgestellt und unter Rühren die 1, 5fache Menge gesättigter Ammoniumsulfat-Lösung langsam zugefügt. Die Suspension bleibt bei 4 °C bis zu drei Tagen stehen bis ein klarer Überstand entstanden ist. Dieser wird abdekantiert, das Präzipitat in der verbleibenden Menge des Überstandes suspendiert und bei >5000 g über 30 Minuten bei Raumtemperatur abzentrifugiert. Sedimente werden in einem Zentrifugenbecher vereinigt ($\Rightarrow$ MAk-Ammoniumsulfatpaste) und anschließend mit Startpuffer gelöst (1 bis 4 Teile Puffer auf 1 Teil Sediment).

Die für einen Protein-A-Lauf errechnete Menge an gelöstem Sediment wird unter Rühren mit einer wässrigen Triton X-100-Lösung, 100 g/l versetzt, so dass die Endkonzentration an Triton X-100 0,5 % beträgt (50 ml Triton X-100-Lösung auf 1000 ml gelöstes Sediment). Der Ansatz wird über einen Spritzenfilter, 0,2 $\mu$m, sterilfiltriert. Der Ansatz wird für 2 bis 18 Stunden bei 4 °C stehen gelassen ($\Rightarrow$ virusinaktive MAk-Lösung).

Anschließend wird sofort die Protein-A Fraktionierung durchgeführt. Das Auftragen der Probe erfolgt unter möglichst aseptischen Bedingungen und mittels einer Pumpe, wobei die Fließgeschwindigkeit dem ein- bis zweifachen Säulenvolumen pro Stunde entsprechen kann, so dass ein Kontakt zwischen MAk und Protein A von mindestens 30 Minuten gewährleistet ist. Nach dem vollständigen Auftragen der Lösungen werden die nicht an Protein-A bindenden Proteine und Triton mit Startpuffer von der Säule gewaschen und so lange gespült, bis die Extinktion/Transmission des Durchflusses den Ausgangswert des Startpuffers wieder erreicht (Dauer: 0,5 bis 3 Stunden). Die Elution des MAk erfolgt mit Elutionspuffer, pH 3,0. Das Eluat wird in einem mit Flockeneis gekühlten Gefäß (gewogene Glasflasche) aufgefangen, in dem ca. 1/10 des Säulenvolumens an 2 M Tris/HCl, pH 8,0 vorgelegt wurde. Die Elution erfolgt so lange, bis der Ausgangswert des Schreibers nahezu wieder erreicht wird ($\Rightarrow$ gereinigte MAk-Lösung).

Nach Bestimmung des Volumens wird unter Rühren 1,5fache Menge gesättigter Ammoniumsulfat-Lösung langsam zugefügt und 60 Minuten gerührt. Anschließend bleibt die Suspension bei 4 °C bis zu drei Tagen stehen bis ein klarer Überstand entstanden ist. Der Ansatz wird aufgeschüttelt und bei >5000 g über 30 Minuten bei Raumtemperatur abzentrifugiert. Der Überstand wird abdekantiert und die Sedimente werden in einem Zentrifugenbecher vereinigt ($\Rightarrow$ MAk-Ammoniumsulfatpaste) und möglichst konzentriert mit Tris/HCl-NaCl-Puffer gelöst (1-3 Teile Puffer auf 1 Teil Sediment).

Die gekläre Proteinlösung wird sofort auf eine regenerierte mit Tris/HCl-NaCl-Puffer, pH 7,5 äquilibrierte Säule mit Sephadex G-25 aufgetragen. Das Volumen der umzusalzenden Proteinlösung soll höchstens 15 % des Säulenvolumens betragen. Die von der Säule kommende Lösung wird über ein Durchflussphotometer und anschließend über einen pH-/Ionenmonitor geleitet. Nachdem die MAk-Lösung vollständig aufgetragen ist, wird die Säule mit Tris/HCl-NaCl-Puffer, pH 7,5 bei einer Fließgeschwindigkeit in der Säule von ca. 20 ml pro $cm^2$ und pro Stunde nachgespült. Der im Ausschlussvolumen der Säule laufende MAk wird in einer sterilen Vorlage entsprechend des UV-Linienschreiber-Profils so lange gesammelt, bis auf dem Linienschreiber annähernd der Ausgangswert der Extinktion/Transmission (ca. 95 %) wieder registriert wird. Der pH-/Ionenmonitor darf noch keine Veränderung der Leitfähigkeit anzeigen ($\Rightarrow$ umgepufferte MAk-Lösung).

Die Leitfähigkeit der Lösung wird kontrolliert und gegebenenfalls durch Zugabe von Natriumchlorid-Lösung, 10 g/l oder Wasser für Injektionszwecke auf die Leitfähigkeit des Puffers eingestellt.

Das Produkt wird auf eine vorbereitete Q-Sepharose-Säule unter möglichst aseptischen Bedingungen mit einer Auftragsgeschwindigkeit von 150 ml pro Stunde aufgegeben (pro ml Q-Sepharose 2 bis 5 mg Protein). Die Elution erfolgt mit Tris/HCl-NaCl-Puffer, pH 7,5 bei einer Fließgeschwindigkeit von ca. 25 ml pro cm$^2$ und pro Stunde. Die von der Säule kommende Lösung wird über ein Durchflussphotometer und anschließend über einen pH-/Ionenmonitor geleitet. Der im Durchfluss von der Säule laufende MAk wird in einer sterilen Vorlage entsprechend des UV-Linienschreiber-Profils so lange gesammelt, bis auf dem Linienschreiber annähernd der Ausgangswert der Extinktion/Transmission (ca. 95 %) wieder registriert wird (⇒ entpyrogenisierte MAk-Lösung).

Der pH-Wert des MAk-haltigen Eluates nach der Anionenaustausch-Chromatographie wird mit 1 N HCl oder 1 N Natriumhydroxid-Lösung auf pH 6,9 eingestellt.

Nach Bestimmung des Volumens wird unter Rühren erneut die 1,5fache Menge gesättigter Ammoniumsulfat-Lösung langsam zugefügt und 60 Minuten gerührt. Anschließend bleibt die Suspension bei 4 °C über Nacht stehen bis ein klarer Überstand entstanden ist. Der Ansatz wird aufgeschüttelt und bei 8525 g über 60 Minuten bei Raumtemperatur abzentrifugiert. Der Überstand wird abdekantiert, die Sedimente in einem Zentrifugenbecher vereinigt (⇒ MAk-Ammoniumsulfatpaste) und möglichst konzentriert mit Natriumphosphat-NaCl-Sorbit-Puffer, pH 7,2 gelöst (1 bis 3 Teile Puffer auf 1 Teil Sediment).

Die geklärte Proteinlösung wird sofort auf eine regenerierte, mit Natriumphosphat-NaCl-Sorbit-Puffer, pH 7,2, äquilibrierte Säule mit Sephadex G-25 unter möglichst aseptischen Bedingungen aufgetragen (höchstens 15 % des Säulenvolumens). Die von der Säule kommende Lösung wird über ein Durchflussphotometer und anschließend über einen pH-/Ionenmonitor geleitet. Die Säule wird mit Natriumphosphat-NaCl-Sorbit-Puffer, pH 7,2 (Fließgeschwindigkeit ca. 20 ml pro cm$^2$ und pro Stunde) nachgespült. Der im Ausschussvolumen der Säule laufende MAk wird in einer sterilen Vorlage entsprechend des UV-Linienschreiber-Profils so lange gesammelt, bis auf dem Linienschreiber annähernd der Ausgangswert der Extinktion/Transmission (ca. 95 %) wieder registriert wird. Der pH-/Ionenmonitor darf noch keine Veränderung der Leitfähigkeit anzeigen (⇒ MAk-Bulklösung, konzentriert).

Der pH-Wert der Lösung wird kontrolliert und mit 1 N HCl oder 1 N Natriumhydroxid-Lösung auf pH 7,2 eingestellt.

Anhand der ermittelten Werte für die Maus-IgG-Konzentration und des Volumens wird der "final bulk" mit Natriumphosphat-NaCl-Sorbit-Puffer, pH 7,2 auf die gewünschte End-Konzentration verdünnt.

Das Produkt wird über einen 0,2 μm-Einmalfilter sterilfiltriert, aliquotiert und der "final bulk" bei -20 °C gelagert.

### Herstellverfahren II

### Anzucht von eukaryontischen Zellen und deren Fermentation in einem Wirbelschichtreaktor

Eine Ampulle aus der Arbeitszellbank, die in flüssigem Stickstoff gelagert ist, wird aufgetaut und die darin befindlichen Zellen werden unter Standard-Zellkulturbedingungen (synthetisches proteinfreies Zellkultur-Medium) in T-Flaschen kultiviert. Nachdem eine Gesamtzellzahl von 6-10 x 10$^7$ Zellen erreicht ist, wird der Inhalt von 4 bis 6 T-Flaschen in einem 500 ml - Spinnergefäß unter Standard-Zellkulturbedingungen weiter kultiviert. Nachdem eine Zellzahl von 1,5-2 x 10$^8$ Zellen erreicht ist, werden die Zellen in 2 Spinnergefäße mit je 1000 ml Volumen transferiert und weiterkultiviert. Nach Erreichen einer Zellzahl von 1,2-1,5 x 10$^9$ Zellen werden die Zellen in einen 900 ml Zellkulturmedium und 250 ml Siran$^R$-Carrier enthaltenden Wirbelschichtreaktor (Bioreaktor Pilot B 500, Papaspyrou Biotechnologie GmbH, Technologiezentrum Jülich, D-52428 Jülich) inokuliert und entsprechend der Handhabungsvorschrift der Fa. Papaspyrou Biotechnologie für 60 Tage bei 36,5 °C kultiviert. Sauerstoffgehalt, pH-Wert, Glukosekonzentration sowie der MAk-Gehalt werden in 1-4tägigen Abständen kontrolliert. Während der Fermentation wird der Zellkulturüberstand kontinuierlich geerntet und bei 4 °C gelagert.

### Proteinchemische Reinigung mit einem "säulenarmen" Verfahren

Nachdem 18 l Zellkulturmedium geerntet sind, wird eine Zellabtrennung mittels "Tangential Flow"-Mikrofiltration gefolgt von einer Sterilfiltration durch einen 0,2 μm-Filter durchgeführt. Die zellfreie und sterile Zellkultur-Ernte wird bei -20 °C gelagert bis genügend Zellkultur-Ernte für die weitere Aufreinigung vorliegt (Zellkultur-Ernte von ca. 250 I Zellkulturmedium). Nach Erreichen der vorgegebenen Batchgröße wird die sterile Zellkultur-Ernte aufgetaut, durch Mikrofiltration geklärt und mittels Ultrafiltration 100 bis 150fach ankonzentriert. Danach wird das Ultrakonzentrat mit einem gleichen Volumen von 2 Mal konzentriertem Startpuffer (pH 8,6) versetzt und sterilfiltriert. Unter sterilen Bedingungen wird eine sterile Triton X-100 Lösung (100 g Triton/l) ad 0,5 % Triton Endkonzentration unter Rühren hinzugegeben. Zur Inaktivierung hüllhaltiger Viren wird die Lösung 4 bis 18 Stunden bei 4 °C stehen gelassen.

Danach wird das mit Triton behandelte Ultrakonzentrat auf eine Protein A-Sepharose-4-Fast-Flow-Säule gepumpt, die zuvor mit 5 Säulenvolumen Startpuffer äquilibriert wurde. Nicht-bindende Proteine und Triton X-100 werden mit 5 Säulenvolumen Startpuffer von der Säule gewaschen. Anschließend wird der an Protein A gebundene MAk mit dem Elutionspuffer (pH 3,0) von der Säule gewaschen und in 130 ml Neutralisationspuffer (pH 8) aufgefangen. Danach wird der pH des in Neutralisationspuffer aufgefangenen MAk bei Bedarf mit NaOH auf pH 7-7,5 eingestellt, mit einem gleichen Volumen 2× Membran-Adsorber Puffer (pH 7;5) verdünnt und sterilfiltriert. Anschließend wird die MAk-Lösung unter sterilen Bedingungen durch einen Membranadsorber gepumpt, der vorher mit WFI und dem Adsorberpuffer (pH 7,5) äquilibriert wurde. Der den MAk enthaltende Durchfluss, der nun von eventuell kontaminierenden Pyrogenen und DNA befreit ist, wird in einem sterilen Behälter gesammelt und mit einem Puffer (pH 7,5) auf 500 μg MAk/ml eingestellt. Danach wird die verdünnte MAk-Lösung durch Ultrafiltration mit einem Viragard Hohlfaser-Modul von potentiell kontaminierenden Viren befreit. Das den virusfreien MAk enthaltende Permeat wird mittels Ultrafiltration auf eine Konzentration von 4-5 mg MAk/ml ankonzentriert und gegen Endpuffer (pH 7,2) diafiltriert. Nach einer Sterilfiltration, einer Verdünnung im Endpuffer und einer erneuten Sterilfiltration wird der MAk als Bulkware abgefüllt.

**Beispiel 3**

**Modifizierter quantitativer Immunreaktivitätstest nach Lindmo**

**Einleitung**

Um den Gehalt immunreaktiver monoklonaler Antikörper in Hybridomüberständen bestimmen zu können, wurde ein Bindungs-Assay mit Antigenüberschuss in Kombination mit einem empfindlichen (1-2 ng Maus-Ig/ml) ELISA-System zur Bestimmung des Anteils ungebundener monoklonaler Antikörper verwendet. Dieser Test hat im Wesentlichen zwei Vorteile gegenüber dem von Lindmo entwickelten Immunreaktivitätstest:

a) Er erlaubt die Bestimmung der Immunreaktivität von sowohl ungereinigten als auch gereinigten nicht-radiomarkierten und radiomarkierten MAk.
b) Er erlaubt die Bestimmung der Immunreaktivität in Abwesenheit unspezifischer Bindung.

**Material**

1.1 Chemikalien und Materialien

| Bezeichnung | Besteller | Bestell-Nr. |
| --- | --- | --- |
| Formaldehyd-Lösung, 37 % | Merck | 818708 |
| Natriumdihydrogenphosphat-1-hydrat | Merck | 6346 |
| di-Natriumhydrogenphosphat-2-hydrat | Merck | 30412 |
| PBS | Behringwerke | |
| Glycin | Merck | 104201 |
| 96 well Mikrotiterplatten, Typ B | Nunc | 4-60445 |
| Ziege-anti-Maus-IgG ("Fänger") | Sigma | M 8642 |
| Tween/PBS für Enzygnost | Behringwerke | OSWE96 |
| Casein | Sigma | C 5890 |
| Ziege-anti-Maus-IgG,-Antikörper, mit alkalischer Phosphatase gekoppelt | SBS | 107-04 |
| 4-Methyl-Umbelliferyl-Phosphat | Sigma | M 8276 |
| SDS | Sigma | L 5750 |

1.2 Geräte

| | | |
| --- | --- | --- |
| Zentrifuge | Heraeus | |
| Minitubes, 1,0 ml | Kühn & Bayer | 64698446 |
| Rotationsgerät | Heidolph | Reo × 2 |
| Analysenwaage | Mettler | DE 100 |
| Magnetrührer | IKA | RCT |
| pH-Meter | WTW | |
| Moulinette | moulinex | |
| Fluoroskan 11 | Merlin | |

**Herstellung der benötigten Lösungen**

4 % Formaldehyd-Lösung nach Lilly

- Zu 100 ml 37 % Formaldehyd-Lösung wird
- 900 ml Aqua bidest. gegeben. In dieser Lösung werden
- 4 g Natriumdihydrogenphosphat und
- 6,5 g di-Natriumhydrogenphosphat unter Rühren gelöst.
- Der pH-Wert der Lösung beträgt pH 7,0.

Waschlösung: 0,05 M Tris-Citrat-Puffer, pH 7,4

- 6,06 g Tris,
- 19,5 g Zitronensäure-1-hydrat und
- 4,25 g Natriumhydroxid
- werden ad 1 l Aqua bidest. gelöst.

Blocklösung: 1 % Casein in PBS, pH 7,2

- 10 g Casein in
- 1 l kaltem PBS, pH 7,2 + Phenolrot
- 30 Minuten rühren lassen,
- anschließend bei 3000 rpm zentrifugieren und
- den Überstand über einen Faltenfilter filtrieren.
- Der pH-Wert der Lösung wird mit NaOH eingestellt.

Substratpuffer: 0,5 M Tris, 0,01 % MgCl, ph 9,6

- 60,57 g Tris und
- 0,1 g Magnesiumchlorid werden
- ad 1 l Aqua bidest. gelöst.

4-Methyl-Umbelliferyl-Phosphat (MUP)-Lösung

- Die Konzentration der MUP-Lösung beträgt 1 mg4-MPU/4, ml Substratpuffer.

Stopp-Lösung: 0,2 M Glycin, 0,2 % SDS, pH 11,7

- 15 g Glycin und
- 2 g SDS werden
- ad 1 l Aqua bidest. gelöst.
- Der pH-Wert der Lösung wird mit 5 N NaOH eingestellt.

**Durchführung des modifizierten quantitativen Immunreaktivitätstest nach Lindmo**

**1. Herstellung des Antigen-enthaltenden Materials ("antigen containing material"; ACM)**

- Gewebe von humanen Tumor-Xenografts (MZ-STO 1, Magenkarzimon), die das Epitop des BW 250/183 exprimieren (auf der Membran von Granulozyten exprimiertes "nonspecific cross reacting antigen" (NCA-95)), werden mit einer Moulinette in 2 bis 5 mm Stücke geschnitten und
- für mindestens 16 Stunden beim Raumtemperatur in 4 % Formaldehyd-Lösung nach Lilly fixiert.
- Nach dem Waschen werden die fixierten Gewebe durch ein rostfreies Edelstahlnetz passiert.
- Die fixierten Zellen werden mindestens 10 Mal in PBS gewaschen bis der Überstand einigermaßen klar ist und anschließend
- 1 Mal in Formalin gewaschen.
- Diese Präparation wird bei 4 °C in Formalin nach Lilly gelagert (1 Teil ACM und 1 Teil Formalin nach Lilly) und als "ACM" bezeichnet.

**2. Bindungs-Assay mit Antigen-Überschuss**

- Das ACM wird mindestens 10 Mal mit PBS gewaschen und anschließend
- wird das Pellet suspendiert und in 100 mM Glycin (4faches Pelletvolumen) für 30 Minuten bei 4 °C inkubiert.
- Danach werden die Zellen noch 4 Mal mit PBS gewaschen.
- Steigende Mengen von ACM (0,1 bis 50 mg) werden in 1 ml Minitubes gegeben und jedes Röhrchen mit 500 $\mu$l Hybridomüberstand, der 25 ng MAk BW 250/183 enthält, über Nacht bei Raumtemperatur inkubiert (Über-Kopf-Rotation).
- Negativ-Kontrollen werden mit 25 ng des anti-Mykoplasmen MAk BW 227/7, der den gleichen Isotyp besitzt (IgG$_1$), inkubiert.
- Das ACM wird abzentrifugiert,
- der Überstand entfernt und
- auf verbleibende Maus-IgG-Moleküle, die nicht an das ACM-Pellet gebunden haben, analysiert.

**3. Bestimmung des Anteils ungebundener Maus-IgG-Moleküle im ELISA-System**

Beschichtung der Mikrotiterplatten mit Antigen

- 96 well-Polystyrol-Mikrotiterplatten werden bei Raumtemperatur mit 50 $\mu$l Ziege-anti-Maus-IgG-Antiserum, 2,5 $\mu$l/ml, pro well über Nacht inkubiert.
- Das Kaninchen-anti-Maus-IgG-Antiserum wird anschließend abgesaugt und die Platten 4 Mal mit 0,05 M Tris-Citrat-Puffer, pH 7,4 gewaschen (ein Waschvorgang = 200 $\mu$l Waschlösung pro well pipettieren und absaugen).
- Die Mikrotiterplatten werden invers auf Zellulose über Nacht getrocknet.
- Mit Trockenpatronen eingeschweißt beträgt die Lagerzeit der so vorbehandelten Mikrotiterplatten mindestens 6 Monate.

Blocken freier Bindungsstellen

- 200 $\mu$l Blocklösung werden pro well pipettiert und die Platten bei Raumtemperatur für 60 Minuten inkubiert.
- Anschließend wird die Blocklösung abgesaugt.

Probenauftrag

- 50 μl der ACM-Überstände, die auf die Anzahl der ungebundenen Maus-IgG-Moleküle analysiert werden sollen, werden pro well aufgetragen und bei Raumtemperatur für 60 Minuten inkubiert.
- Danach werden die Mikrotiterplatten 3 Mal wie oben beschrieben mit Waschlösung gewaschen.

Amplifikation und Nachweis

- 50 μl eines mit alkalischer Phosphatase gekoppelten Ziege-anti-Maus-IgG-Antikörpers, 1:250 verdünnt, werden pro well aufgetragen und bei Raumtemperatur für 30 Minuten inkubiert.
- Die Mikrotiterplatten werden 3 Mal mit Waschlösung wie oben beschrieben gewaschen.
- 50 μl MUP-Lösung wird pro well aufgetragen und bei Raumtemperatur für 30 Minuten inkubiert.
- Die Substratreaktion wird nach Inkubation bei Raumtemperatur für 30 Minuten durch Zugabe von 100 μl Stopp-Lösung gestoppt.
- Anschließend wird die Fluoreszenz gemessen: die Anregungswellenlänge beträgt 355 nm und die Emissionswellenlänge 460 nm.

Die Empfindlichkeit dieses Tests liegt im Bereich von 1 bis 2 ng Maus-IgG/ml.

Mathematische Bestimmung der Immunreaktivität

$$IR[\%] = 100\ \%-[100\ \% \times (\text{MAk-Konz. im Überstand/MAk-Ausgangskonz.})]$$

Die Berechnung der Immunreaktivität erfolgt am Ablesepunkt. Dieser Punkt liegt bei demjenigen ACM-Volumen (X-Achse der ELISA-Verlaufskurve), bei dem gerade noch kein Abfall des Plateauwertes der unspezifischen Kontrolle (unspezifische Bindung) vorhanden ist.

**Beispiel 4**

**Szintigramme, aufgenommen nach Injektion von Tc-99m-markiertem MAk BW 250/183**

Die für die Markierung mit Tc-99m verwendeten MAk-Chargen unterscheiden sich aufgrund des Herstellungsverfahrens in ihrer Immunreaktivität. Die Daten sind in der nachstehenden Tabelle 10 zusammengefasst:

| MAk-Charge | Herstellungsverfahren | Immunreaktivität | Szintigramm |
|---|---|---|---|
| MAk BW 250/183 | konventionelle Fermentation und konventionelles Reinigungsverfahren | 70 - 80 % | Abbildung GRAN 11 (Figur 3) Abbildung GRAN 21 (Figur 4) |
| MAk BW 250/183 | Wirbelschichtreaktor-Fermentation und "säulenarmes" Reinigungsverfahren | >90 % | Abbildung GRAN 91 (Figur 5) Abbildung GRAN 81 (Figur 6) Abbildung GRAN 71 (Figur 7) |

**Patentansprüche**

1. Präparation von immunreaktiven Proteinen zur therapeutischen oder diagnostischen Verwendung, **dadurch gekennzeichnet,**

**dass** der in einem modifizierten Lindmo-Test gemäß Beispiel 3 bestimmte prozentuale Anteil an immunreaktiven Molekülen > 90% bezüglich der Gesamtzahl von Molekülen aus immunreaktiven und nichtimmunreaktiven, proteinchemisch nicht unterschiedlichen Molekülen ist, wobei es sich bei dem immunreaktiven Protein um einen monoklonalen, chimärisierten oder humanisierten Antikörper handelt, der an ein Epitop auf CD66 bindet.

2. Präparation nach Anspruch 1,
**dadurch gekennzeichnet,**
**dass** die immunreaktiven Proteine eine Markierung, insbesondere eine radioaktive Markierung ausgewählt aus $^{99m}$Tc, $^{186}$Re, $^{188}$Re, $^{90}$Y und Astatin tragen.

3. Präparation nach einem der Ansprüche 1 bis 2,
**dadurch gekennzeichnet,**
**dass** die immunreaktiven Proteine durch Herstellung in Zellkultur, insbesondere durch Expression in rekombinanten eukaryontischen Wirtszellen, erzeugt wurden.

4. Präparation nach einem der Ansprüche 1 bis 3,
**dadurch gekennzeichnet,**
**dass** es sich bei CD66 um CD66 a, b, c, e und bevorzugt um CD 66 e handelt.

5. Präparation nach einem der Ansprüche 1 bis 4,
**dadurch gekennzeichnet,**
**dass** es sich bei dem immunreaktiven Protein um einen monoklonalen Antikörper handelt, ausgewählt aus BW 431/26 und BW 250/183.

6. Verfahren zur Herstellung einer Präparation von immunreaktiven Proteinen zur therapeutischen oder diagnostischen Verwendung,
**dadurch gekennzeichnet,**
**dass** es eine Wirbelschichtreaktor-Fermentation einer das immunreaktive Protein exprimierenden Wirtszelle in einem geeigneten Kulturmedium und die Gewinnung des Proteins aus der Wirtszelle und/oder aus dem Kulturmedium umfasst, wobei der in einem modifizierten Lindmo-Test gemäß Beispiel 3 bestimmte prozentuale Anteil an immunreaktiven Molekülen >81 % bezüglich der Gesamtzahl von Molekülen aus immunreaktiven und nichtimmunreaktiven, proteinchemisch nicht unterschiedlichen Molekülen ist, wobei es sich bei dem immunreaktiven Protein um einen monoklonalen, chimärisierten oder humanisierten Antikörper handelt, der an ein Epitop auf CD66, auf CD45, auf N-CAM, auf FVIIIRAG und/oder auf dem VEGF/VEGF-Rezeptor-Komplex bindet, und der weder an VEGF noch an den VEGF-Rezeptor bindet.

7. Verfahren nach Anspruch 6,
**dadurch gekennzeichnet,**
**dass** man eine eukaryontische Wirtszelle verwendet.

8. Verfahren nach Anspruch 6 oder 7,
**dadurch gekennzeichnet,**
**dass** das immunreaktive Protein durch eine säulenarme Reinigungsprozedur aus der Wirtszelle und/oder dem Kulturmedium gewonnen wird, wobei als säulenchromatographischer Schritt nur eine Affinitätschromatographie durchgeführt wird.

9. Verfahren nach Anspruch 6 oder 7,
**dadurch gekennzeichnet,**
**dass** das immunreaktive Protein durch eine konventionelle Reinigungsprozedur aus der Wirtszelle und/oder dem Kulturmedium gewonnen wird, wobei als säulenchromatographische Schritte
eine Affinitätschromatographie,
eine Gelchromatographie und
eine Anionaustausch-Chromatographie
durchgeführt werden.

10. Verfahren nach einem der Ansprüche 6 bis 9,
**dadurch gekennzeichnet,**
**dass** der in einem modifizierten Lindmo-Test gemäß Beispiel 3 bestimmte prozentuale Anteil an immunreaktiven Molekülen > 90 % bezüglich der Gesamtzahl von Molekülen aus immunreaktiven und nichtimmunreaktiven, proteinchemisch nicht unterschiedlichen Molekülen ist, wobei es sich bei dem immunreaktiven Protein um einen monoklonalen, chimärisierten oder humanisierten Antikörper handelt, der an ein Epitop auf CD66, auf CD45, auf N-CAM, auf FVIIIRAG und/oder auf dem VEGF/VEGF-Rezeptor-Komplex bindet, und der weder an VEGF noch an den VEGF-Rezeptor bindet.

11. Verfahren nach Anspruch 9,
**dadurch gekennzeichnet,**
**dass** die Präparation entsprechend dem Herstellungsverfahren I hergestellt wird, umfassend die Schritte:

- Ernte des Zellkulturmediums und Zellabtrennung,
- Ankonzentrierung,
- Lösung, Virusdeaktivierung mittels Detergenz-Behandlung, Sterilfiltration,
- Affinitätschromatographie,
- Ankonzentrierung,
- Lösung, Umpufferung mittels Gelchromatographie,
- Anionenaustausch-Chromatographie,
- Ankonzentrierung,
- Lösung, Umpufferung mittels Gelchromatographie,
- Einstellung der gewünschten Endkonzentration des Bulks,
- Sterilfiltration.

12. Verfahren nach Anspruch 8,
**dadurch gekennzeichnet,**
**dass** die Präparation entsprechend dem Herstellungsverfahren II hergestellt wird, umfassend die Schritte:

- Ernte des Zellkulturmediums, Zellabtrennung, Sterilfiltration und Lagerung,
- Auftauen, Ernte, gefolgt von Mikrofiltration und Ultrafiltration,
- Verdünnung,
- Behandlung mit Detergenz,
- Affinitätschromatographie,
- Verdünnung und Sterilfiltration,
- Anionenaustausch-Membranadsorption,
- Virusfiltration mittels Ultrafiltration,
- Ultrafiltration und Diafiltration,
- Filtration und Verdünnung,
- Sterilfiltration.

13. Verfahren nach einem der Ansprüche 6 bis 12,
**dadurch gekennzeichnet,**
**dass** es sich bei dem immunreaktiven Protein um einen monoklonalen Antikörper handelt, ausgewählt aus BW 431/26, BW 250/183, YTH 24.5 und BW 278/105.

14. Verwendung von Protein-Präparationen nach einem der Ansprüche 1 bis 5 als Träger von γ-Strahlern, wie z.B. Tc-99m, zur Herstellung eines Mittels für die Diagnose entzündlicher Prozesse und von das Knochenmark verdrängenden Prozessen, beispielsweise Metastasen von Prostata-Karzinomen, Mamma-Karzinomen und Lymphomen.

15. Verwendung von Protein-Präparationen nach einem der Ansprüche 1 bis 5 als Träger von α- und β-Strahlern zur Herstellung eines Mittels für die Therapie von Erkrankungen des hämatopoietischen Systems, vorzugsweise von Leukämien.

**Claims**

1. Preparation of immunoreactive proteins for therapeutic or diagnostic use,
**characterized in that**
the percentage of immunoreactive molecules determined in a modified Lindmo test according to example 3 is > 90 % relative to the total number of molecules consisting of immunoreactive and non-immunoreactive molecules that do not differ in their protein chemistry wherein the immunoreactive protein is a monoclonal, chimerized or humanized antibody which binds to an epitope on CD66.

2. Preparation according to claim 1,
   **characterized in that**
   the immunoreactive proteins carry a label and in particular a radioactive label selected from $^{99m}Tc$, $^{186}Re$, $^{188}Re$, $^{90}Y$ and astatine.

3. Preparation according to one of the claims 1 to 2,
   **characterized in that**
   the immunoreactive proteins are obtained by production in cell culture, in particular by expression in recombinant eukaryotic host cells.

4. Preparation according to one of the claims 1 to 3,
   **characterized in that**
   the CD66 is CD66 a, b, c, e and preferably CD66 e.

5. Preparation according to one of the claims 1 to 4,
   **characterized in that**
   the immunoreactive protein is a monoclonal antibody selected from BW 431/26 and BW 250/183.

6. Process for producing a preparation of immunoreactive proteins for therapeutic or diagnostic use,
   **characterized in that**
   it comprises a fluidized bed reactor fermentation of a host cell expressing the immunoreactive protein in a suitable culture medium and isolating the protein from the host cell and/or from the culture medium, wherein the percentage of immunoreactive molecules determined in a modified Lindmo test according to example 3 is > 81 % relative to the total number of molecules consisting of immunoreactive and non-immunoreactive molecules that do not differ in their protein chemistry, wherein the immunoreactive protein is a monoclonal, chimerized or humanized antibody which binds to an epitope on CD66, on CD45, on N-CAM, on FVIIIRAG and/or on the VEGF/VEGF receptor complex and does not bind to VEGF nor to the VEGF receptor.

7. Process according to claim 6,
   **characterized in that**
   a eukaryotic host cell is used.

8. Process according to claim 6 or 7,
   **characterized in that**
   the immunoreactive protein is isolated from the host cell and/or the culture medium by a column-reduced purification procedure in which only one affinity chromatography is carried out as the column chromatographic step.

9. Process according to claim 6 or 7,
   **characterized in that**
   the immunoreactive protein is isolated from the host cell and/or the culture medium by conventional purification procedure in which an affinity chromatography, a gel chromatography and an anion exchange chromatography are carried out as column chromatographic steps.

10. Process according to one of the claims 6 to 9,
    **characterized in that**
    the percentage of immunoreactive molecules determined in a modified Lindmo test according to example 3 is > 90 % relative to the total number of molecules consisting of immunoreactive and non-immunoreactive molecules that do not differ in their protein chemistry wherein the immunoreactive protein is a monoclonal, chimerized or humanized antibody which binds to an epitope on CD66, on CD45, on N-CAM, on FVIIIRAG and/or on the VEGF/VEGF receptor complex and does not bind to VEGF nor to the VEGF receptor.

11. Process according to claim 9,
    **characterized in that**
    the preparation is produced according to the production process I comprising the steps:

- harvesting the cell culture medium and cell separation,
- concentrating,
- dissolution, virus inactivation by means of detergent treatment, sterile filtration,
- affinity chromatography,
- concentration,
- dissolution, rebuffering by means of gel chromatography,
- anion exchange chromatography,
- concentration,
- dissolution, rebuffering by means of gel chromatography,
- adjusting the desired final concentration of the bulk,
- sterile filtration.

**12.** Process according to claim 8,
**characterized in that**
the preparation is produced according to the production process I comprising the steps:

- harvesting the cell culture medium, cell separation, sterile filtration and storage,
- thawing, harvesting followed by microfiltration and ultrafiltration,
- dilution,
- treating with detergent,
- affinity chromatography,
- dilution and sterile filtration,
- anion exchange membrane adsorption,
- virus filtration by means of ultrafiltration,
- ultrafiltration and diafiltration,
- filtration and dilution,
- sterile filtration.

**13.** Process according to one of the claims 6 to 12,
**characterized in that**
the immunoreactive protein is a monoclonal antibody selected from BW 431/26, BW 250/183, YTH 24.5 and BW 278/105.

**14.** Use of protein preparations according to one of the claims 1 to 5 as carriers of γ emitters such as e.g. Tc-99m to produce an agent for the diagnosis of inflammatory processes and bone marrow displacing processes, for example metastases of prostate carcinomas, mamma carcinomas and lymphomas.

**15.** Use of protein preparations according to one of the claims 1 to 5 as carriers of α and β emitters to produce an agent for the treatment of diseases of the haematopoetic system, preferably of leukaemia.

**Revendications**

**1.** Préparation de protéines immunoréactives destinée à une application thérapeutique ou diagnostique, **caractérisée en ce que** la fraction en pourcentage de molécules immunoréactives, déterminée par un test de Lindmo modifié selon l'exemple 3, est > 90 % par rapport au nombre total de molécules parmi des molécules immunoréactives et non immunoréactives, non différentes du point de vue de la chimie des protéines, la protéine immunoréactive étant un anticorps monoclonal, chimérisé ou humanisé qui se lie à un épitope sur un CD66.

**2.** Préparation selon la revendication 1, **caractérisée en ce que** les protéines immunoréactives portent un marquage, en particulier un marquage radioactif choisi parmi $^{99m}$Tc, $^{186}$Re, $^{188}$Re, $^{90}$Y et l'astatine.

**3.** Préparation selon l'une des revendications 1 à 2, **caractérisée en ce que** les protéines immunoréactives sont obtenues par préparation en culture de cellules, en particulier par expression dans des cellules hôtes eucaryotes recombinantes.

**4.** Préparation selon l'une des revendications 1 à 3, **caractérisée en ce que** le CD66 est un CD66 a, b, c, e et de préférence un CD66 e.

**EP 1 276 770 B1**

5. Préparation selon l'une des revendications 1 à 4, **caractérisée en ce que** la protéine immunoréactive est un anticorps monoclonal choisi parmi BW 431/26 et BW 250/183.

6. Procédé de production d'une préparation de protéines immunoréactives destinée à une application thérapeutique ou diagnostique, **caractérisé en ce qu'**il comprend une fermentation dans un réacteur à lit fluidisé d'une cellule hôte exprimant la protéine immunoréactive dans un milieu de culture approprié et la récupération de la protéine à partir de la cellule hôte et/ou du milieu de culture, la fraction en pourcentage de molécules immunoréactives, déterminée par un test de Lindmo modifié selon l'exemple 3, étant > 81 % par rapport au nombre total de molécules parmi des molécules immunoréactives et non immunoréactives, non différentes du point de vue de la chimie des protéines, la protéine immunoréactive étant un anticorps monoclonal, chimérisé ou humanisé qui se lie à un épitope sur un CD66, sur un CD45, sur un N-CAM, sur un FVIIIRAG et/ou sur le complexe VEGF/récepteur de VEGF, et qui ne se lie ni au VEGF, ni au récepteur de VEGF.

7. Procédé selon la revendication 6, **caractérisé en ce que** l'on utilise une cellule hôte eucaryote.

8. Procédé selon la revendication 6 ou 7, **caractérisé en ce que** l'on obtient la protéine immunoréactive à partir de la cellule hôte et/ou du milieu de culture par une procédure de purification utilisant peu de colonnes, en n'effectuant comme étape de chromatographie sur colonne qu'une chromatographie d'affinité.

9. Procédé selon la revendication 6 ou 7, **caractérisé en ce que** l'on obtient la protéine immunoréactive à partir de la cellule hôte et/ou du milieu de culture par une procédure de purification classique en effectuant comme étapes de chromatographie sur colonne:

une chromatographie d'affinité,
une chromatographie sur gel et
une chromatographie par échange d'anions.

10. Procédé selon l'une des revendications 6 à 9, **caractérisé en ce que** la fraction en pourcentage de molécules immunoréactives, déterminée par un test de Lindmo modifié selon l'exemple 3, est > 90 % par rapport au nombre total de molécules parmi des molécules immunoréactives et non immunoréactives, non différentes du point de vue de la chimie des protéines, la protéine immunoréactive étant un anticorps monoclonal, chimérisé ou humanisé qui se lie à un épitope sur un CD66, sur un CD45, sur un N-CAM, sur un FVIIIRAG et/ou sur le complexe VEGF/récepteur de VEGF, et qui ne se lie ni au VEGF, ni au récepteur de VEGF.

11. Procédé selon la revendication 9, **caractérisé en ce que** la préparation est produite selon le procédé de production I, comprenant les étapes:

- récolte du milieu de culture et séparation des cellules,
- concentration,
- dissolution, déactivation des virus par traitement avec un détergent, filtration stérile,
- chromatographie d'affinité,
- concentration,
- dissolution, changement de tampon par chromatographie sur gel,
- chromatographie par échange d'anions,
- concentration,
- dissolution, changement de tampon par chromatographie sur gel,
- ajustement de la concentration finale désirée du mélange,
- filtration stérile.

12. Procédé selon la revendication 8, **caractérisé en ce que** la préparation est produite selon le procédé de préparation II, comprenant les étapes:

- récolte du milieu de culture, séparation des cellules, filtration stérile et stockage,
- décongélation, récolte, suivie d'une microfiltration et d'une ultrafiltration,
- dilution,
- traitement avec un détergent,
- chromatographie d'affinité,
- dilution et filtration stérile,

- adsorption sur membrane échangeuse d'anions,
- filtration des virus par ultrafiltration,
- ultrafiltration et diafiltration,
- filtration et dilution,
- filtration stérile.

13. Procédé selon l'une des revendications 6 à 12, **caractérisé en ce que** la protéine immunoréactive est un anticorps monoclonal choisi parmi BW 431/26, BW 250/183, YTH 24.5 et BW 278/105.

14. Utilisation de préparations de protéines selon l'une des revendications 1 à 5 comme supports d'émetteurs de rayons γ comme, par exemple, Tc-99m, pour la préparation d'un agent destiné au diagnostic de processus inflammatoires et de processus de suppression de la moelle osseuse, par exemple de métastases de cancers de la prostate, de cancers du sein et de lymphomes.

15. Utilisation de préparations de protéines selon l'un des revendications 1 à 5 comme support d'émetteurs de rayons α et β pour la préparation d'un agent destiné à la thérapie de maladies du système hématopoïétique, de préférence de leucémies.

Figur 1:

**Figur 2A:**

**Figur 2B:**

**Figur 2C:**

**Figur 2D:**

**Figur 2E:**

**Fig. 3**

LT   POST   RT                    RT   ANT.   LT

**Fig. 4**

RT   ANT   LT         LT   POST   RT

**Fig. 5**

**Fig. 6**

3 hours        22 hours

31

**Fig. 7**

**EP 1 276 770 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5132405 A, Houston
- US 4873316 A, Meade
- WO 8605202 A
- EP 0727480 A
- WO 9622310 A
- DE 19744531

**In der Beschreibung aufgeführte Nicht-Patentliteratur**

- **STAHL et al.** *PNAS,* 1976, vol. 73, 4045-4049
- **SEITZ et al.** *European Journal of Nuclear Medicine,* 1999, vol. 26, 1265
- **JAGODA et al.** *Journal of Immunological Methods,* 1994, vol. 173, 191-201
- **MATTES, M.J.** *Int. J. Cancer,* 1995, vol. 61, 286-288
- **MORALES et al.** *Nuclear Medicine & Biology,* 1999, vol. 26, 275-279
- **BOVEN et al.** *Blood,* 1986, vol. 67 (2), 429-435
- **BRUYNCK et al.** *Br. J. Cancer,* 1993, vol. 67, 436-440
- **WAIBEL et al.** *Nature Biotechnology,* 1999, vol. 17, 897-901
- **SCHUBIGER et al.** *Euro J. Nucl. Med.,* 1989, vol. 15, 605-608
- *Eur. J. Nucl. Med.,* 1988, vol. 14, 523-528
- *Int. J. Cancer,* 1985, vol. 36, 75-84
- *J. Histochem. Cytochem.,* 1986, vol. 34, 209-214
- *Pediatr. Hematol. Oncol.,* 1989, vol. 6, 73-83
- *J. Immunol.,* 1985, vol. 134, 3056-3061
- Leucocyte Typing III: White Cell Differentiation Antigens. Oxford University Press, 788-803
- *British Journal of Cancer,* 1992, vol. 645, 234-238
- White Cell Differentiation Antigens. Oxford University Press, 788-803
- *British J. Cancer,* 1992, vol. 645, 234-238
- *J. Nucl. Med.,* 1987, vol. 28, 721
- **VISSER et al.** *J. Nucl. Med.,* 1993, vol. 34, 1953-1963
- **GRIFFITH et al.** *Cancer Res.,* 1991, vol. 51, 4594-4602
- **DENORA et al.** *Anticancer Res.,* 1997, vol. 17, 1735-1744